# EUROPÄISCHE PATENTSCHRIFT

(11) **EP 3 090 722 B1**
(45) Veröffentlichungstag und Bekanntmachung des Hinweises auf die Patenterteilung: **09.01.2019**
(21) Anmeldenummer: 15166847.2
(22) Anmeldetag: 07.05.2015
(51) Int. Cl.: A61K 6/00, A61K 6/083

(54) **DURCH KETTENÜBERTRAGUNGSPOLYMERISATION HÄRTBARE DENTALMATERIALIEN**
DENTAL MATERIALS WHICH CAN BE HARDENED BY CHAIN-TRANSFER POLYMERIZATION
MATÉRIAU DENTAIRE DURCISSABLE PAR POLYMÉRISATION AVEC RÉACTION DE TRANSFERT

(43) Veröffentlichungstag der Anmeldung: 09.11.2016
(73) Patentinhaber: Ivoclar Vivadent AG, 9494 Schaan (LI); Technische Universität Wien, 1040 Wien (AT)
(72) Erfinder: Moszner, Norbert, 9495 Triesen (LI); Lamparth, Iris, 9472 Grabs (CH); Fischer, Urs-Karl, 9320 Arbon (CH); Rist, Kai, 6800 Feldkirch (AT); Burtscher, Peter, A-6830 Rankweil (AT); Liska, Robert, 2123 Schleinbach (AT); Gorsche, Christian, Dr., 1140 Wien (AT); Seidler, Konstanze, 2105 Oberrohrbach (AT)
(74) Vertreter: Uexküll & Stolberg

(56) Entgegenhaltungen:
- US-A- 2 667 469
- US-A- 2 694 699
- DATABASE CA [Online] CHEMICAL ABSTRACTS SERVICE, COLUMBUS, OHIO, US; 1986, KAWAGUCHI, TOSHIO ET AL: "Film-forming adhesives for teeth and bones", XP002750529, gefunden im STN Database accession no. 105:214120 & JP S61 151104 A (TOKUYAMA SODA CO., LTD., JAPAN) 9. Juli 1986 (1986-07-09)

## Beschreibung

Die vorliegende Erfindung betrifft thermisch- und/oder lichthärtende radikalische polymerisierbare Materialien, die sich besonders als Dentalwerkstoffe eignen.

Radikalische Polymerisate, werden durch radikalische Polymerisation von einem (Homopolymerisat) oder mehreren radikalisch polymerisierbaren Monomeren (Copolymerisat) gebildet. Dabei werden je nach der Funktionalität der polymerisierten Monomeren lineare (monofunktionelle Monomere) oder vernetzte (di- oder multifunktionelle Monomere) Polymere erhalten.

Radikalische Polymerisationen lassen sich bekanntlich in Substanz (Bulkpolymerisation), Lösung, Suspension oder Emulsion durchführen. Zur Polymerisationsauslösung werden radikalbildende Initiatoren zugesetzt, die durch Thermolyse, Photolyse oder Redoxreaktion Radikale bilden. Die radikalische Polymerisation läuft nach einem Kettenwachstumsmechanismus ab, bei dem sich die polymerisationsauslösenden Radikale, die sog. Primärradikale, an die Doppelbindung der Monomeren addieren. Die so gebildeten Startradikale addieren in einer schnellen Wachstumsreaktion viele weitere Monomermoleküle bis das Wachstum der Polymerradikale durch Kombination oder Disproportionierung abgebrochen wird und sich die fertigen Makromoleküle bilden.

Bei der radikalischen Polymerisation kommt es häufig zu Kettenübertragungen. Hierbei überträgt das Polymerradikal im Austausch gegen ein anderes Atom ein Elektron auf ein zweites Molekül, z.B. auf ein Monomer-, Lösungsmittel- oder Polymermolekül. Das dabei neu gebildete Radikal kann wieder eine Polymerisation auslösen. Durch die Zugabe von Kettenüberträgern, sog. Kettenreglern, lässt sich die zahlenmittlere Molmasse des Polymers gezielt regulieren (vgl. H. G. Elias, Makromoleküle, Bd. 1,6 Aufl., Wiley-VCH, Weinheim etc. 199, 299-352). Zu den bekannten Kettenüberträgern zählen beispielsweise die Mercaptane, die durch Übertragung eines H-Atoms Thiyl-Radikale bilden, welche dann eine neue Polymerisationssequenz initiieren.
Als Kettenüberträger haben sich doppelbindungshaltige Reagenzien besonders bewährt, die nach einem radikalischen Addition-Fragmentierung-Kettenübertragungs-Mechanismus, engl. "**a**ddition-**f**ragmentation **c**hain **t**ransfer" (AFCT) reagieren. Als AFCT-Reagenzien sind Schwefelverbindungen, wie Allylsulfide, Allylsulfone, Dithioester, Dithiocarbamate, Xanthate und Trithiocarbonate besonders wirksam und gut untersucht (vgl. G. Moad, E. Rizzardo, S. H. Thang, Polymer 49 (2008) 1079-1131). Darüber hinaus sind **r**eversible AFCT-Reagenzien (RAFT-Reagenzien), wie z.B. Dithioester, Dithiocarbamate, Trithiocarbonate oder Xanthate, aus der kontrollierten radikalischen Polymerisation bekannt (vgl. z.B. Moad et al., aaO).
Die US 2,694,699 beschreibt die Homo- und Copolymerisation von *α*-Sulfonyloxyacrylaten. Als Kettenregler können Alkylmercaptane zugesetzt werden.
Die US 5,932,675 offenbart ein Verfahren zur Herstellung von Polymeren mit geringem Molekulargewicht durch radikalische Polymerisation, wobei die Kontrolle des Molekulargewichts durch die Zugabe z.B. von α-(t-Butanthiomethyl)styrol als Kettenübertragungsreagenz

Die JP S61 151 104 offenbart ein adhesives Material auf der Basis von Polymere, die phosphorhaltige Gruppen aufweisen. Die Langzeitstabilität des Materials wird durch Zugabe eines Chelatbildners verbessert.

Die Verwendung der bekannten übertragungsaktiven Verbindungen ermöglichst eine Kontrolle des Molekulargewichts der Polymere, hat jedoch den Nachteil, dass sie zu einer deutlichen Reduktion der Polymerisationsgeschwindigkeit führt. Die Verwendung von **α**-Sulfonyloxyacrylaten, Alkyl- oder Arylsulfonsäurevinylestern als Kettenübertragungsreagenzien ist nicht bekannt.

Der Erfindung liegt die Aufgabe zugrunde, Dentalmaterialien und insbesondere Beschichtungsmaterialien, Prothesenmaterialien, Adhäsive und Komposite bereitzustellen, die sich im Vergleich zu bekannten Materialien auf Basis multifunktioneller (Meth)acrylate nach der Härtung durch einen engeren Bereich des Glasüberganges, eine verbesserte Schlagzähigkeit und eine verringerte Polymerisationsschrumpfungskraft bei ähnlichen mechanischen Eigenschaften auszeichnen. Die Materialien sollen vor allem eine hohe Reaktivität und Aushärtungsgeschwindigkeit, keine Eigenfarbe und keinen störenden Geruch ausweisen.

Diese Aufgabe wird erfindungsgemäß durch radikalisch polymerisierbare Zusammensetzungen gelöst, die mindestens einen Sulfonsäureester der Formel 1 enthalten: 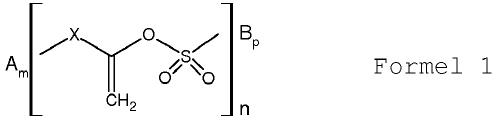 in der die Variablen die folgenden Bedeutungen haben:
- A: H, CN, ein cycloaliphatischer, linearer oder verzweigter aliphatischer C₁-C₃₀-Kohlenwasserstoffrest, der durch einen oder mehrere, vorzugsweise 1 bis 6, besonders bevorzugt 1 bis 3 Substituenten, vorzugsweise -CH₃, -C₂H₅, -OH, -OCH₃ und/oder -O-COCH₃, substituiert sein kann, der durch eine oder mehrere, vorzugsweise 1 bis 4, besonders bevorzugt 1 bis 2 Urethangruppen, Estergruppen, 0 und/oder S unterbrochen sein kann und der 1 bis 4, vorzugsweise 1 oder 2 Benzolgruppen enthalten kann, ein aromatischer C₆-C₃₀-Kohlenwasserstoffrest, der durch eine oder mehrere, vorzugsweise 1 bis 6, besonders bevorzugt 1 bis 3 Substituenten, vorzugsweise -CH₃, -C₂H₅, -OH, -OCH₃ und/oder -O-COCH₃, substituiert sein kann, oder eine Kombination davon;
- X: -COO-, -CON (R¹) - oder entfällt, wobei die Bindung an A über 0 oder N erfolgt;
- B: ein cycloaliphatischer, linearer oder verzweigter aliphatischer C₁-C₃₀-Kohlenwasserstoffrest, der durch einen oder mehrere, vorzugsweise 1 bis 6, besonders bevorzugt 1 bis 3 Substituenten, vorzugsweise C₁- bis C₅-Alkyl, -OH, -O-COCH₃ und/oder C₁- bis C₅-Alkoxy, substituiert sein kann, der durch eine oder mehrere, vorzugsweise 1 bis 4, besonders bevorzugt 1 bis 2 Urethangruppen, Estergruppen, 0 und/oder S unterbrochen sein kann und der 1 bis 4, vorzugsweise 1 oder 2 Benzolgruppen enthalten kann, ein aromatischer C₆-C₃₀-Kohlenwasserstoffrest, der durch einen oder mehrere, vorzugsweise 1 bis 6, besonders bevorzugt 1 bis 3 Substituenten, vorzugsweise C₁- bis C₅-Alkyl, -OH, -O-COCH₃ und/oder C₁- bis C₅-Alkoxy, substituiert sein kann,
oder eine Kombination davon;
- R¹: Wasserstoff ein cycloaliphatischer, linearer oder verzweigter aliphatischer C₁-C₁₀-Kohlenwasserstoffrest, der durch eine oder mehrere, vorzugsweise 1 bis 4, besonders bevorzugt 1 bis 2 Sauerstoffatome unterbrochen sein kann und der durch eine oder mehrere, vorzugsweise 1 bis 4, besonders bevorzugt 1 bis 2 OH-Gruppen substituiert sein kann, oder
ein aromatischer C₆-C₁₀-Kohlenwasserstoffrest, der durch eine oder mehrere, vorzugsweise 1 bis 4, besonders bevorzugt 1 bis 2 OH-Gruppen substituiert sein kann;
- m: eine ganze Zahl von 1 bis 6;
- n: eine ganze Zahl von 1 bis 6;
- p: eine ganze Zahl von 1 bis 6; wobei
m und p nicht gleichzeitig größer als 1 sein können und wobei dann, wenn m = 1 ist, p = n ist, und wenn p = 1 ist, m = n ist.

Die Formel erstreckt sich nur auf solche Verbindungen, die mit der chemischen Valenzlehre vereinbar sind. Beispielsweise kann m dann, wenn A ein C₁-Rest ist, maximal 4 sein. Der Hinweis, dass ein Rest durch eine oder mehrere Urethangruppen, O-Atome, S-Atome etc. unterbrochen ist, ist so zu verstehen, dass diese Gruppen jeweils in die Kohlenstoffkette des Restes eingeschoben werden. Diese Gruppen sind damit beidseitig durch C-Atome begrenzt und können nicht endständig sein. C₁-Reste können nicht unterbrochen sein. Der Hinweis, dass ein Rest eine Benzolgruppe enthält, bedeutet demgegenüber, dass diese Gruppe auch endständig sein kann, wobei ggf. verbleibende yl-Stellen durch H abgesättigt sind. Unter Kombinationen werden Gruppen verstanden, die sich aus den jeweils angegebenen Bedeutungen zusammensetzen, beispielsweise aus aromatischen und aliphatischen Resten, wie z.B. -Ph-CH₂-Ph-, oder aus mehreren aromatischen Resten, wie z.B. -Ph-Ph-, oder aus aromatischen und/oder aliphatischen Resten und anderen der genannten Gruppen, wie z.B. -Ph-O-Ph- (Ph=Phenyl).

Die Verbindungen der Formel 1 sind bei der radikalischen Polymerisation als Kettenüberträger aktiv und deren Verwendung als Kettenregler ist ebenfalls Gegenstand der Erfindung.

Die Formel 1 ist so zu verstehen, dass n der in Klammern stehenden Gruppe an den Rest A oder den Rest B gebunden sind. Im ersten Fall ist m gleich 1 und p gleich n. Die Formel 1 lässt sich in diesem Fall zu Formel 2 vereinfachen:

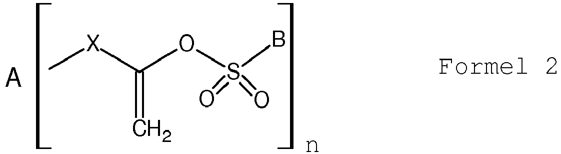

In Formel 2 sind 1 bis 6 der in Klammen stehenden Gruppe jeweils an A gebunden. A ist in diesem Fall ein n-wertiger Rest und B ein einwertiger Rest. Im Fall der Formel 2 haben die Variablen vorzugsweise die folgenden Bedeutungen:
- A: H, CN, ein aliphatischer, linearer oder verzweigter C₁-C₃₀-Kohlenwasserstoffrest, der durch eine oder mehrere, vorzugsweise 1 bis 4, besonders bevorzugt 1 bis 2 Substituenten, die aus CH₃, -C₂H₅, -OH, -OCH₃ und/oder -O-COCH₃ ausgewählt sind, substituiert sein kann, der durch eine oder mehrere, vorzugsweise 1 bis 4, besonders bevorzugt 1 bis 2 Urethangruppen, Estergruppen, 0 und/oder S unterbrochen sein kann und der 1 bis 4, vorzugsweise 1 oder 2 Benzolgruppen, vorzugsweise 1,4-Phenylengruppen, enthalten kann, ein aromatischer C₆-C₃₀-Kohlenwasserstoffrest, der durch eine oder mehrere, vorzugsweise 1 bis 4, besonders bevorzugt 1 bis 2 Substituenten, die aus CH₃, -C₂H₅, -OH, -OCH₃ und/oder -O-COCH₃ ausgewählt sind, substituiert sein kann, oder eine Kombination davon;
- X: -COO-, -CON(R¹)- oder entfällt, wobei die Bindung an A über 0 oder N erfolgt, und wobei A vorzugsweise entfällt, wenn A ein aromatischer Kohlenwasserstoffrest oder CN ist;
- B: ein aliphatischer, linearer oder verzweigter C₁-C₂₀-Kohlenwasserstoffrest, der durch einen oder mehrere, vorzugsweise 1 bis 3, besonders bevorzugt 1 bis 2 Substituenten, vorzugsweise C₁- bis C₅-Alkyl und/oder C₁- bis C₅-Alkoxy, substituiert sein kann, der durch eine oder mehrere, vorzugsweise 1 bis 4, besonders bevorzugt 1 bis 2 Urethangruppen, Estergruppen, 0 oder S unterbrochen sein kann und der 1 bis 4, vorzugsweise 1 oder 2 Benzolgruppen, vorzugweise 1,4-Phenylengruppen, enthalten kann, ein aromatischer C₆-C₁₈-Kohlenwasserstoffrest, der durch einen oder mehrere, vorzugsweise 1 bis 3, besonders bevorzugt 1 bis 2 Substituenten, vorzugsweise C₁- bis C₅-Alkyl und/oder C₁- bis C₅-Alkoxy, substituiert sein kann, oder eine Kombination davon;
- R¹: Wasserstoff oder ein linearer oder verzweigter aliphatischer C₁-C₁₀-Kohlenwasserstoffrest, der durch eine oder mehrere, vorzugsweise 1 bis 4, besonders bevorzugt 1 bis 2 Sauerstoffatome unterbrochen sein kann und der durch eine oder mehrere, vorzugsweise 1 bis 4, besonders bevorzugt 1 bis 2 OH-Gruppen substituiert sein kann;
- n: eine ganze Zahl von 1 bis 6.

Besonders bevorzugt sind Verbindungen der Formel 2, in der die Variablen die folgenden Bedeutungen haben:
- A: ein aliphatischer linearer oder verzweigter C₁-C₂₀-Kohlenwasserstoffrest, der durch eine oder mehrere, vorzugsweise 1 bis 4, besonders bevorzugt 1 bis 2 1,4-Phenylengruppen, Urethangruppen, Estergruppen, 0 und/oder S unterbrochen sein kann und der durch eine oder mehrere, vorzugsweise 1 bis 4, besonders bevorzugt 1 bis 2 OH-Gruppen substituiert sein kann, oder ein Phenylrest, der durch einen oder mehrere, vorzugsweise 1 bis 3, besonders bevorzugt 1 bis 2 Substituenten, vorzugsweise -CH₃, und/oder -OCH₃ substituiert sein kann;
- X: -O-CO- oder entfällt, wobei die Bindung an A über 0 erfolgt und wobei A vorzugsweise entfällt, wenn A ein aromatischer Kohlenwasserstoffrest oder CN ist;
- B: ein aliphatischer linearer oder verzweigter C₁-C₂₀-Kohlenwasserstoffrest, der durch eine oder mehrere, vorzugsweise 1 bis 4, besonders bevorzugt 1 bis 2 Sauerstoffatome unterbrochen sein kann und der 1 bis 4, vorzugsweise 1 oder 2 Benzolgruppen, vorzugsweise 1,4-Phenylengruppen, enthalten kann, oder ein Phenylrest, der durch 1 oder 2, vorzugsweise 1 -OCH₃ oder vorzugsweise -CH₃ Gruppe(n) substituiert sein kann;
- n: 1 oder 2.

Ganz besonders bevorzugt sind Verbindungen der Formel 2, in der die Variablen die folgenden Bedeutungen haben:
- A: ein aliphatischer linearer oder verzweigter C₁-C₁₂-Kohlenwasserstoffrest, der durch 1 bis 2 Urethangruppen, Estergruppen, 0 und/oder S unterbrochen sein kann, der 1 bis 2 Benzolgruppen, vorzugsweise 1,4-Phenylengruppen, enthalten kann und der durch 1 bis 2 OH-Gruppen substituiert sein kann, oder ein Phenylrest, der durch -CH₃, und/oder -OCH₃ substituiert sein kann;
- X: -O-CO- oder entfällt, wobei die Bindung an A über 0 erfolgt und wobei A vorzugsweise entfällt, wenn A ein aromatischer Kohlenwasserstoffrest oder CN ist;
- B: ein aliphatischer linearer oder verzweigter C₁-C₆-Kohlenwasserstoffrest, der durch ein Sauerstoffatom unterbrochen sein kann und eine Benzolgruppe, vorzugsweise eine 1,4-Phenylengruppe, enthalten kann, ein Phenylrest oder pMethylphenylrest;
- n: 1 oder 2.

Insbesondere sind solche Verbindungen der Formel 2 bevorzugt, in der die Variablen die folgenden Bedeutungen haben:
- A: ein aliphatischer linearer C₁-C₈-Kohlenwasserstoffrest, der durch 1 bis 2 Urethangruppen oder 0 unterbrochen sein kann, oder ein Phenylrest;
- X: -O-CO-, wobei die Bindung an A über 0 erfolgt und wobei A vorzugsweise entfällt, wenn A ein aromatischer Kohlenwasserstoffrest oder CN ist;
- B: ein aliphatischer linearer C₁-C₃-Kohlenwasserstoffrest, ein Phenylrest oder p-Methylphenylrest;
- n: 1 oder 2.

Wenn mehrere der in Klammern stehenden Gruppe an den Rest B gebunden sind, ist p gleich 1 und m gleich n. Die Formel 1 lässt sich in diesem Fall zu Formel 3 vereinfachen:

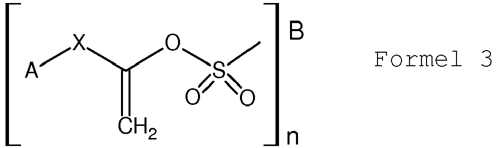

In Formel 3 sind 1 bis 6 der in Klammen stehenden Gruppe jeweils an B gebunden. A ist in diesem Fall ein einwertiger Rest und B ein n-wertiger Rest. Im Fall der Formel 3 haben die Variablen vorzugsweise die folgenden Bedeutungen:
- A: Wasserstoff, -CN, ein Phenylrest, der durch einen oder mehrere, vorzugsweise 1 bis 3, besonders bevorzugt 1 bis 2 Substituenten, vorzugsweise -CH₃, -C₂H₅, -OH, -OCH₃ und/oder -O-COCH₃, substituiert sein kann, oder ein aliphatischer linearer oder verzweigter C₁-C₂₀-Kohlenwasserstoffrest, der durch eine oder mehrere, vorzugsweise 1 bis 4, besonders bevorzugt 1 bis 2 Urethangruppen, Estergruppen, 0 und/oder S unterbrochen sein kann, der 1 bis 4, vorzugsweise 1 oder 2 Benzolgruppen, vorzugsweise 1,4-Phenylengruppen, enthalten kann und der durch eine oder mehrere, vorzugsweise 1 bis 4, besonders bevorzugt 1 bis 2 OH-Gruppen substituiert sein kann;
- B: ein cycloaliphatischer, linearer oder verzweigter aliphatischer C₁-C₂₀-Kohlenwasserstoffrest, der durch eine oder mehrere, vorzugsweise 1 bis 4, besonders bevorzugt 1 bis 2 OH-Gruppen substituiert sein kann, der durch eine oder mehrere, vorzugsweise 1 bis 4, besonders bevorzugt 1 bis 2 Urethangruppen, Estergruppen, 0 und/oder S unterbrochen sein kann und der 1 bis 4, vorzugsweise 1 oder 2 Benzolgruppen enthalten kann, ein aromatischer C₆-C₁₈-Kohlenwasserstoffrest, der durch eine oder mehrere, vorzugsweise 1 bis 4, besonders bevorzugt 1 bis 2 OH-Gruppen substituiert sein kann, oder eine Kombination davon;
- X: -COO- oder -CON(R¹)- oder entfällt, wobei die Bindung an A über 0 oder N erfolgt und wobei A vorzugsweise entfällt, wenn A ein aromatischer Kohlenwasserstoffrest oder CN ist;
- R¹: Wasserstoff oder ein linearer oder verzweigter aliphatischer C₁-C₁₀-Kohlenwasserstoffrest, der durch eine oder mehrere, vorzugsweise 1 bis 4, besonders bevorzugt 1 bis 2 Sauerstoffatome unterbrochen sein kann und der durch eine oder mehrere, vorzugsweise 1 bis 4, besonders bevorzugt 1 bis 2 OH-Gruppen substituiert sein kann;
- n: eine ganze Zahl von 2 bis 4.

Besonders bevorzugt sind Verbindungen der Formel 3, in der die Variablen die folgenden Bedeutungen haben:
- A: H, ein Phenylrest, der durch einen oder mehrere Substituenten, vorzugsweise -CH₃, -OH, -OCH₃, substituiert sein kann, oder ein aliphatischer, linearer oder verzweigter C₁-C₈-Kohlenwasserstoffrest, der durch eine oder mehrere, vorzugsweise 1 bis 4, besonders bevorzugt 1 bis 2 Urethangruppen, Estergruppen und/oder 0 unterbrochen sein kann;
- B: ein aliphatischer, linearer oder verzweigter C₁-C₁₀-Kohlenwasserstoffrest, der durch eine oder mehrere, vorzugsweise 1 bis 4, besonders bevorzugt 1 bis 2 O-Atome unterbrochen sein kann und der 1 bis 4, vorzugsweise 1 oder 2 Benzolgruppen, vorzugsweise 1,4-Phenylengruppen, enthalten kann,
ein aromatischer C₆-C₁₀-Kohlenwasserstoffrest, oder eine Kombination davon;
- X: -COO- oder entfällt, wobei die Bindung an A über 0 erfolgt und wobei A vorzugsweise entfällt, wenn A ein aromatischer Kohlenwasserstoffrest oder CN ist; und
- n: 2.

Ganz besonders bevorzugt sind Verbindungen der Formel 3, in der die Variablen die folgenden Bedeutungen haben:
- A: H, ein Phenylrest, der durch eine -CH₃, -OH, -OCH₃, substituiert sein kann, oder ein aliphatischer, linearer oder verzweigter C₁-C₈-Kohlenwasserstoffrest, der durch eine oder mehrere, vorzugsweise 1 bis 4, besonders bevorzugt 1 bis 2 Urethangruppen, Estergruppen und/oder 0 unterbrochen sein kann;
- B: ein aliphatischer, linearer oder verzweigter C₁-C₄-Kohlenwasserstoffrest, der durch 1 bis 2 O-Atome unterbrochen sein kann und der 1 bis 2 Benzolgruppen, vorzugsweise 1,4-Phenylengruppen, enthalten kann, ein aromatischer C₆-C₁₀-Kohlenwasserstoffrest, vorzugsweise Phenyl oder Naphthyl, oder eine Kombination davon, vorzugsweise -Ph-CH₂-Ph-; -Ph-O-Ph- oder -Ph-Ph-;
- X: -COO- oder entfällt, wobei die Bindung an A über 0 erfolgt und wobei A vorzugsweise entfällt, wenn A ein aromatischer Kohlenwasserstoffrest oder CN ist; und
- n: 2.

Insbesondere sind solche Verbindungen der Formel 3 bevorzugt, in der die Variablen die folgenden Bedeutungen haben:
- A: ein Phenylrest oder ein aliphatischer linearer oder verzweigter C₁-C₃-Alkylrest;
- B: ein aliphatischer, linearer C₁-C₄-Kohlenwasserstoffrest, der ein O-Atom unterbrochen sein kann und der eine Benzolgruppe, vorzugsweise 1,4-Phenylengruppe, enthalten kann, Phenyl, -Ph-Ph-, -Ph-CH₂-Ph-, -Ph-O-Ph- oder Naphthyl;
- X: -COO- oder entfällt, wobei die Bindung an A über 0 erfolgt; und
- n: 2.

Der Einfachheit halber werden hierin die Bezeichnungen für einwertige Reste, wie Phenyl und Naphthyl, auch für mehrwertige Reste mit mehr als einer yl-Stelle verwendet, wobei sich die jeweilige Bedeutung aus den Formeln 1, 2 und 3 ergibt. Phenyl (Ph) schließt damit insbesondere Phenylen und Benzol-1,3,5-triyl ein. Naphthyl steht vorzugsweise für einen Naphthalin-2,6-diyl-Rest.

Polymerisationsübertragungsaktive Verbindungen der Formel 1 sind zum Teil bereits bekannt und nach bekannten Synthesemethoden herstellbar. Beispielsweise lassen sich durch Umsetzung von Brenztraubensäurederivaten mit Sulfonyl-Halogeniden unter basischen Bedingungen erfindungsgemäße polymerisationsübertragungsaktiven Verbindungen der Formel 1 erhalten:

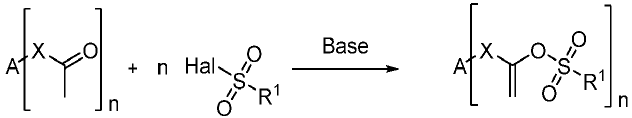

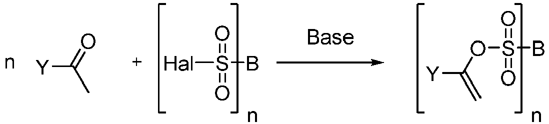

Ein konkretes Beispiel ist:

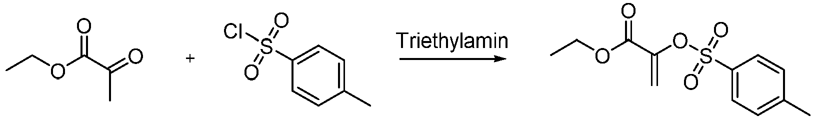

Bevorzugte Beispiele für die erfindungsgemäßen polymerisationsübertragungsaktiven Verbindungen der Formel 1 sind:

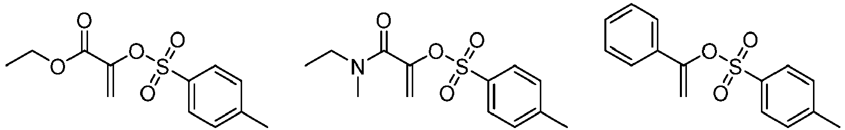

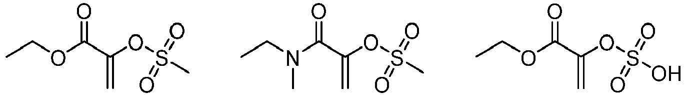

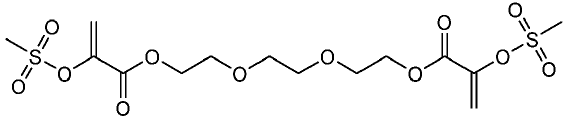

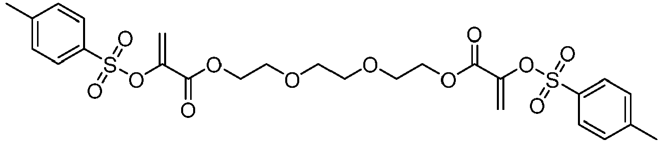

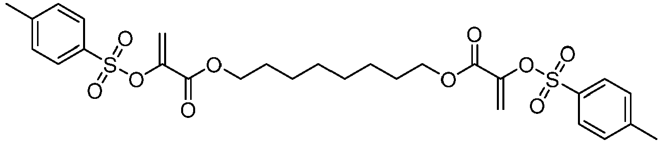

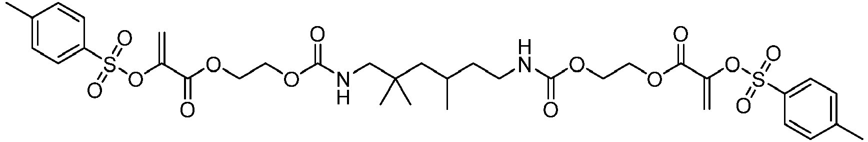

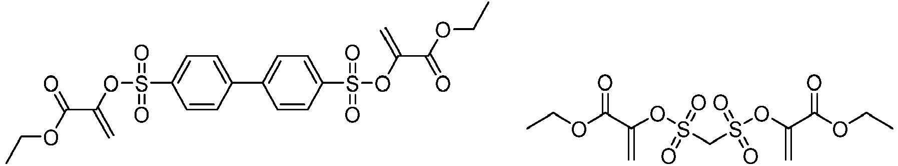

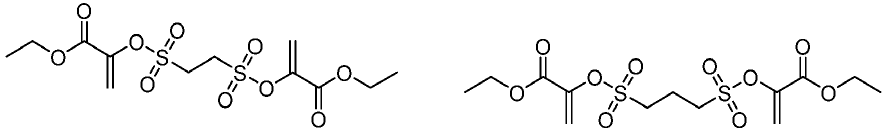

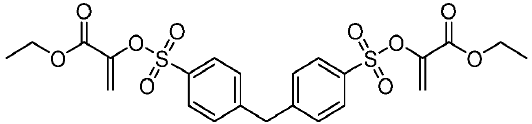

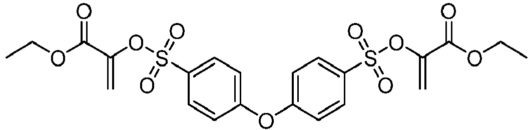

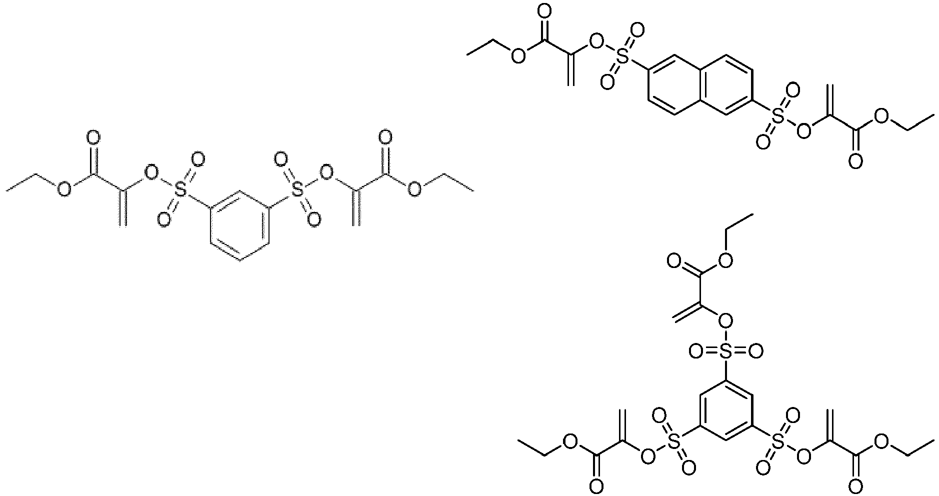

Die erfindungsgemäßen Verbindungen der Formel 1 lassen sich vorteilhaft als Kettenüberträger zur Kontrolle und Steuerung der Netzwerkstruktur bei der Polymerisation von Mono(meth)acrylaten, multifunktionellen (Meth)acrylaten und deren Mischungen einsetzen. Kettenüberträger werden hierin auch als Übertragungsreagenzien oder Regler bezeichnet. Im Vergleich zu reinen (Meth)acrylaten ergeben sie Polymernetzwerke mit einem engeren Glasübergang, d.h. der Glasübergang findet in einem engeren Temperaturbereich statt. Außerdem werden homogenere Polymernetzwerke erhalten, d.h. Netzwerke, die dadurch charakterisiert sind, dass sie eine engere Verteilung der Molmasse zwischen den Vernetzungspunkten aufweisen. Dies hat den Vorteil, dass Kettenspannungen durch Relaxationsprozesse besser abgebaut werden können und dass z.B. im Bereich von Dentalzementen ein schnelleres Debonding-on-Demand (DoD) bewirkt werden kann.

Außerdem wurde überraschend gefunden, dass die Verbindungen der Formel 1 bei der Polymerisation von (Meth)acrylaten die Glasübergangstemperatur der polymerisierten Materialien deutlich verringern ohne die Polymerisationsgeschwindigkeit nennenswert abzusenken. Eine verringerte Glasübergangstemperatur hat den Vorteil, dass die Polymeren bei niedrigeren Temperaturen erweicht werden können. Dies gestattet z.B. im Fall von Adhäsiven und Zementen ein gezieltes Lösen der Klebeverbindung (Debonding-on-Demand).

Darüber hinaus zeichnen sich die erhaltenen Polymermaterialien durch eine verbesserte Schlagzähigkeit aus, was z.B. bei stereolithographisch hergestellten Formkörpern oder bei dentalen Prothesen von großem Vorteil ist.

Die Verbindungen der Formel 1 bewirken bei der vernetzenden Polymerisation z.B. von multifunktionellen (Meth)acrylaten eine deutliche Verzögerung der Gelbildung und gewährleisten damit eine längere Gelzeit, d.h., dass sich das 3-dimensionale Polymernetzwerk erst später ausbildet. Die verlängerte Gelzeit wirkt sich günstig auf die Polymerisationsschrumpfungsspannung (PKS) aus, weil innere Spannungen länger durch Fließprozesse ausglichen werden können. Dies führt zu deutlich geringeren Schrumpfspannungen, was z.B. bei komplizierten Geometrien von Formteilen oder dentalen Füllungskompositen von großem Vorteil ist.

Die Verbindungen der Formel 1 können daher zur Verringerung der Glasübergangstemperatur, zur Verringerung der Polymerisationsschrumpfungskraft und/oder der Verbesserung der Schlagzähigkeit von Polymerisaten verwendet werden. Die Polymerisationsschrumpfungskraft wird auch als Polymerisationsschrumpfungsspannung bezeichnet.

Erfindungsgemäß enthalten solche Dentalwerkstoffe neben einer Verbindung der Formel 1 zusätzlich mindestens ein radikalisch polymerisierbares multifunktionelles (Meth) acrylat oder eine Mischung von mono- und multifunktionellen (Meth) acrylaten und mindestens einen Initiator für die radikalische Polymerisation
Das Molverhältnis zwischen den ethylenischen Unsättigungen in den radikalisch polymerisierbaren Monomeren und den Sulfonatgruppierungen in den als Regler dienenden Sulfonsäureestern der Formeln 1 bis 3 beträgt gemäß vorliegender Erfindung vorzugsweise zumindest 2:1 und in besonders bevorzugten Ausführungsformen zumindest 3:1, noch bevorzugter zumindest 5:1 oder zumindest 10:1, um die Kettenlängen durch die Gegenwart des Reglers nicht zu stark zu reduzieren. Bezogen auf das Molekulargewicht werden vorzugsweise 50 bis 99 Gew.-%, noch bevorzugter 60 bis 98 Gew.-%, noch bevorzugter 70 bis 95 Gew.-%, der ethylenisch ungesättigten Monomere, bezogen auf das Gesamtgewicht der Monomere und Regler, eingesetzt, was einerseits von den Substituenten A und B in den Formeln 1 bis 3 und andererseits von den jeweils zu polymerisierenden Monomeren abhängt.
Besonders bevorzugt sind Werkstoffe, die als radikalisch polymerisierbares Monomer mindestens ein multifunktionelles (Meth)acrylat oder eine Mischungen von mono- und multifunktionellen (Meth)acrylaten enthalten. Unter monofunktionellen (Meth)acrylaten werden Verbindungen mit einer, unter polyfunktionellen (Meth)acrylaten Verbindungen mit zwei oder mehr, vorzugsweise 2 bis 4 radikalisch polymerisierbaren Gruppen verstanden. Gemäß einer ganz besonders bevorzugten Ausführungsform enthalten die erfindungsgemäßen Zusammensetzungen mindestens ein Dimethacrylat oder eine Mischung von Mono- und Dimethacrylaten. Materialien, die als radikalisch polymerisierbares Monomer mono- und multifunktionelle (Meth)acrylaten enthalten, eignen sich besonders als Dentalwerkstoffe, wobei für Materialien, die intraoral gehärtet werden, Methacrylate bevorzugt sind.

Beispiele für besonders geeignete mono- oder multifunktionelle (Meth)acrylate sind Methyl-, Ethyl-, 2-Hydroxyethyl-, Butyl-, Benzyl-, Tetrahydrofurfuryl- oder Isobornyl(meth)acrylat, p-Cumyl-phenoxyethylenglycolmethacrylat (CMP-1E), Bisphenol-A-di(meth)acrylat, Bis-G(M)A (ein Additionsprodukt aus (Meth)acrylsäure und Bisphenol-A-diglycidylether), ethoxy- oder propoxyliertes Bisphenol-A-di(meth)acrylat, wie z.B. das Bisphenol-A-dimethacrylat SR-348c (Sartomer) mit 3 Ethoxygruppen oder 2,2-Bis[4-(2-(meth)acryloxypropoxy)phenyl]propan, UD(M)A (ein Additionsprodukt aus 2-Hydroxyethyl(meth)acrylat und 2,2,4- oder 2,4,4-Trimethylhexamethylen-1,6-diisocyanat), Di-, Tri- oder Tetraethylenglycoldi(meth)acrylat, Trimethylolpropantri(meth)acrylat, Pentaerythrittetra(meth)acrylat, sowie Glycerindi- und tri(meth)acrylat, 1,4-Butandioldi(meth)-acrylat, 1,10-Decandioldi(meth)acrylat (D₃MA), 1,12-Dodecandioldi(meth)acrylat oder oligomere Polyether-, Polyester-, Epoxy-, Silikon- oder Urethan-(meth)acrylate.

Geeignet sind außerdem auch thermo- oder Photolabile Di(meth)-acrylate, wie z.B. das Additionsprodukt aus 2 mol 2-Acetoacetoxyethylmethacrylat und 1 mol 2,2,4- oder 2,4,4-Trimethylhexamethylen-1,6-diisocyanat (thermolabil) oder Methacrylsäure-2-[2-(4-{2-methyl-2-[2-(methacryloyloxy)-ethylcarbamoyloxy]-propionyl}-phenoxy)-ethoxycarbonylamino]-ethylester. Mischungen von thermo- oder Photolabilen Monomeren und Verbindungen der Formel 1 eignen sich besonders für Werkstoffe mit Debonding-on-Demand-Eigenschaften.

Zur Initiierung der radikalischen Photopolymerisation werden vorzugsweise Benzophenon, Benzoin sowie deren Derivate oder α-Diketone oder deren Derivate, wie 9,10-Phenanthrenchinon, 1-Phenyl-propan-1,2-dion, Diacetyl oder 4,4'-Dichlorbenzil, eingesetzt. Besonders bevorzugt werden Campherchinon (CQ) und 2,2-Dimethoxy-2-phenyl-acetophenon und ganz besonders bevorzugt α-Diketone in Kombination mit Aminen als Reduktionsmittel, wie z.B. 4-(Dimethylamino)-benzoesäureester (EDMAB), N,N-Dimethylaminoethylmethacrylat, N,N-Dimethyl-sym.-xylidin oder Triethanolamin, verwendet. Gut geeignet sind auch Norrish-Typ-I-Photoinitiatoren, vor allem Acyl- der Bisacylphosphinoxide, und insbesondere Monoacyltrialkyl- bzw. Diacyldialkylgermanium-Verbindungen, wie z.B. Benzoyltrimethylgermanium, Dibenzoyldiethylgermanium oder Bis(4-methoxybenzoyl)diethylgermanium (MBDEGe). Dabei lassen sich vorteilhaft auch Mischungen der verschiedenen Photoinitiatoren einsetzen, wie z.B. Bis(4-methoxybenzoyl)diethylgermanium in Kombination mit Campherchinon und 4-Dimethylaminobenzoesäureethylester.

Als Initiatoren für die Heißhärtung eignen sich besonders thermische Initiatoren, wie Azoverbindungen, z.B. Azobisisobutyronitril, oder Peroxide, z.B. Dibenzoylperoxid sowie Benzpinakol und 2,2'-Dialkylbenzpinakole. Als Initiatoren für eine bei Raumtemperatur durchgeführte Polymerisation werden vorzugsweise Redox-Initiatorkombinationen ("Redoxinitiator"), wie z.B. Kombinationen von Benzoylperoxid mit N,N-Dimethylsym.-xylidin oder N,N-Dimethyl-p-toluidin, verwendet. Darüber hinaus sind auch Redoxsysteme bestehend aus Peroxiden und solchen Reduktionsmitteln, wie z.B. Ascorbinsäure, Barbituraten oder Sulfinsäuren, besonders geeignet.

Gemäß einer bevorzugten Ausführungsform enthalten die erfindungsgemäßen Werkstoffe, insbesondere Dentalwerkstoffe, zusätzlich organischen oder vorzugsweise anorganischen partikulären Füllstoff, besonders bevorzugt einen oder mehrere anorganische partikuläre Füllstoffe. Mischungen die Monomere, vorzugsweise multifunktionelle (Meth)acrylate, Mischungen davon oder Mischungen von multifunktionellen und monofunktionellen (Meth)acrylaten, und Füllstoffe enthalten, werden als Komposite bezeichnet.

Besonders geeignet sind Füllstoffe auf der Basis von Oxiden mit einer Partikelgröße von 0,010 bis 15 µm, wie SiO₂, ZrO₂ und TiO₂ bzw. Mischoxiden aus SiO₂, ZrO₂, ZnO und/oder TiO₂, nanopartikuläre oder mikrofeine Füllstoffe mit einer Partikelgröße von 10 bis 300 nm, wie pyrogene Kieselsäure oder Fällungskieselsäure sowie Glaspulver mit einer Partikelgröße von 0,01 bis 15 µm, vorzugweise von 0,2 bis 1,5 µm, wie Quarz-, Glaskeramik- oder röntgenopake Glaspulver von z.B. Barium- oder Strontiumaluminiumsilikatgläsern, und röntgenopake Füllstoffe mit einer Partikelgröße von 0,2 bis 5 µm, wie Ytterbiumtrifluorid, Tantal(V)-oxid, Bariumsulfat oder Mischoxide von SiO₂ mit Ytterbium(III)-oxid oder Tantal(V)-oxid. Auch faserförmige Füllstoffe, Nanofasern oder Whiskers sind nicht ausgeschlossen. Wenn nicht anders angegeben handelt es sich bei allen Partikelgrößen um gewichtsmittlere Partikelgrößen.

Die Füllstoffe werden nach der Partikelgröße unterteilt in Makrofüller und Mikrofüller. Makrofüller werden durch Mahlen von Quarz, röntgenopaken Gläsern, Borosilikaten oder von Keramik gewonnen, sind rein anorganischer Natur und bestehen meist aus splitterförmigen Teilen. Bevorzugt sind Makrofüller mit einer mittleren Partikelgröße von 0,2 bis 10 µm. Als Mikrofüller werden vorzugsweise pyrogenes SiO₂ oder Fällungskieselsäure eingesetzt, oder auch Mischoxide, z.B. SiO₂-ZrO₂, die durch hydrolytische Cokondensation von Metallalkoxiden zugänglich sind. Die Mikrofüller haben vorzugsweise eine mittlere Partikelgröße von ca. 5 bis 100 nm.

Zur Verbesserung des Verbundes zwischen den Füllstoffpartikeln und der vernetzten Polymerisationsmatrix können SiO₂-basierende Füllstoffe mit (Meth)acrylat-funktionalisierten Silanen oberflächenmodifiziert werden. Ein Beispiel für solche Silane ist 3-(Meth) acryloyloxypropyltrimethoxysilan. Zur Oberflächenmodifizierung von nichtsilikatischen Füllstoffen z.B. von ZrO₂ oder TiO₂ können auch funktionalisierte saure Phosphate, wie z.B. 10-(Meth) acryloyloxydecyldihydrogenphosphat eingesetzt werden.

Der Füllungsgrad richtet sich nach dem gewünschten Anwendungszweck. Füllungskomposite haben vorzugsweise einen Füllstoffgehalt von 75-90 Gew.-% und Kompositzemente von 50-75 Gew.-%.

Gegebenenfalls können die erfindungsgemäß eingesetzten Zusammensetzungen weitere Additive enthalten, vor allem Stabilisatoren, Farbmittel, mikrobiozide Wirkstoffe, fluoridionenabgebende Additive, Treibmittel, optische Aufheller, Weichmacher oder UV-Absorber.

Die erfindungsgemäßen Dentalmaterialien enthalten vorzugsweise 0,5 bis 40 Gew.-%, bevorzugt 2,0 bis 30 Gew.-% und besonders bevorzugt 5,0 bis 30 Gew.-% mindestens einer Verbindung der allgemeinen Formel 1.

Zusätzlich enthalten die Dentalmaterialien vorzugsweise auch 0,01 bis 5,0 Gew.-%, bevorzugt 0,1 bis 5,0 Gew.-% und besonders bevorzugt 0,1 bis 3, 0 Gew.-% Initiator (en) für die radikalische Polymerisation, besonders bevorzugt einen Photoinitiator, und ganz besonders bevorzugt auch 5 bis 80 Gew.-%, bevorzugt 10 bis 70 Gew.-% und besonders bevorzugt 10 bis 60 Gew.-% multifunktionelle (s) (Meth)acrylat(e).

Weiterhin enthalten die erfindungsgemäßen Dentalmaterialien vorzugsweise 0 bis 90 Gew.-%, bevorzugt 0 bis 80 Gew.-% und besonders bevorzugt 0 bis 70 Gew.-% Füllstoff (e), wobei der Füllstoffgehalt wie oben beschrieben auf die geplante Verwendung der Werkstoffe abgestimmt wird.

Die erfindungsgemäßen Dentalwerkstoffe können in Abhängigkeit vom gewünschten Einsatzzweck vorteilhaft auch ein Lösungsmittel enthalten, vorzugsweise 0 bis 80 Gew.-%, besonders bevorzugt 0 bis 60 Gew.-% und ganz besonders bevorzugt 0 bis 40 Gew.-% Lösungsmittel. Bevorzugte Lösungsmittel sind Wasser, Ethanol, Polyethylenglycol und Mischungen daraus.

Außerdem können die erfindungsgemäßen Dentalmaterialien weitere(s) Additiv(e) enthalten, vorzugsweise in einer Menge von 0 bis 5 Gew.-%, besonders bevorzugt 0 bis 3 Gew.-% und ganz besonders bevorzugt 0,2 bis 3 Gew.-%.

Dabei sind erfindungsgemäß Dentalmaterialien besonders bevorzugt, welche die folgenden Komponenten enthalten:
(a) 0,5 bis 40 Gew.-%, bevorzugt 1,0 bis 30 Gew.-% und besonders bevorzugt 2,0 bis 30 Gew.-% mindestens einer Verbindung der allgemeinen Formel 1,
(b) 0,01 bis 5,0 Gew.-%, bevorzugt 0,1 bis 5,0 Gew.-% und besonders bevorzugt 0,1 bis 3,0 Gew.-% Initiator(en),
(c) 5 bis 80 Gew.-%, bevorzugt 10 bis 70 Gew.-% und besonders bevorzugt 10 bis 60 Gew.-% multifunktionelle(s) (Meth)acrylat(e).

Die Dentalmaterialien enthalten vorzugsweise außerdem:
(d) 0 bis 50 Gew.-%, bevorzugt 0 bis 40 Gew.-% und besonders bevorzugt 0 bis 30 Gew.-% monofunktionelle(s) (Meth)-acrylat(e),
(e) 0 bis 95 Gew.-%, bevorzugt 5 bis 85 Gew.-% und besonders bevorzugt 5 bis 80 Gew.-% Füllstoff(e),
(f) 0 bis 5 Gew.-%, bevorzugt 0 bis 3 Gew.-% und besonders bevorzugt 0,2 bis 3 Gew.-% Additiv(e) und
(g) 0 bis 80 Gew.-%, bevorzugt 0 bis 60 Gew.-% und besonders bevorzugt 0 bis 40 Gew.-% Lösungsmittel, insbesondere Wasser, Ethanol und/oder Polyethylenglycol.

Dentalwerkstoffe zur Verwendung als Prothesenmaterialien, Adhäsive oder Beschichtungsmaterialien enthalten vorzugsweise:
(a) 0,5 bis 40 Gew.-%, bevorzugt 1,0 bis 30 Gew.-% und besonders bevorzugt 2,0 bis 30 Gew.-% mindestens einer Verbindung der allgemeinen Formel 1,
(b) 0,01 bis 5,0 Gew.-%, bevorzugt 0,1 bis 5,0 Gew.-% und besonders bevorzugt 0,1 bis 3,0 Gew.-% Initiator(en),
(c) 5 bis 80 Gew.-%, bevorzugt 10 bis 80 Gew.-% und besonders bevorzugt 15 bis 80 Gew.-% multifunktionelle(s) (Meth)-acrylat(e),
(d) 0 bis 40 Gew.-%, bevorzugt 0 bis 30 Gew.-% und besonders bevorzugt 0 bis 20 Gew.-% monofunktionelle(s) (Meth)-acrylat(e),
(e) 0 bis 5 Gew.-%, bevorzugt 0 bis 3 Gew.-% und besonders bevorzugt 0,2 bis 3 Gew.-% Additiv(e).
(f) 0 bis 60 Gew.-%, bevorzugt 0 bis 50 Gew.-% und besonders bevorzugt 0 bis 40 Gew.-% Lösungsmittel, vorzugsweise Wasser, Ethanol und/oder Polyethylenglycol.

Dentalwerkstoffe zur Verwendung als Komposite, insbesondere als Füllungskomposite und Zemente, enthalten vorzugsweise:
(a) 0,5 bis 30 Gew.-%, bevorzugt 1,0 bis 30 Gew.-% und besonders bevorzugt 2,0 bis 20 Gew.-% mindestens einer Verbindung der allgemeinen Formel 1,
(b) 0,01 bis 5,0 Gew.-%, bevorzugt 0,1 bis 5,0 Gew.-% und besonders bevorzugt 0,1 bis 3,0 Gew.-% Initiator(en),
(c) 5 bis 50 Gew.-%, bevorzugt 10 bis 40 Gew.-% und besonders bevorzugt 10 bis 30 Gew.-% multifunktionelle(s) (Meth)-acrylat(e),
(d) 0 bis 30 Gew.-%, bevorzugt 0 bis 20 Gew.-% und besonders bevorzugt 0 bis 10 Gew.-% monofunktionelle(s) (Meth)-acrylat(e),
(e) 5 bis 85 Gew.-%, bevorzugt 20 bis 85 Gew.-% und besonders bevorzugt 30 bis 80 Gew.-% Füllstoff(e), und
(f) 0 bis 5 Gew.-%, bevorzugt 0 bis 3 Gew.-% und besonders bevorzugt 0,2 bis 3 Gew.-% Additiv(e).

Dentalwerkstoffe zur Herstellung dentaler Formkörper und Restaurationen durch 3D-Druck, enthalten vorzugsweise:
(a) 0,5 bis 50 Gew.-%, bevorzugt 0,5 bis 30 Gew.-% und besonders bevorzugt 1,0 bis 20 Gew.-% mindestens einer Verbindung der allgemeinen Formel 1,
(b) 0,001 bis 2,0 Gew.-%, bevorzugt 0,01 bis 2,0 Gew.-% und besonders bevorzugt 0,05 bis 1,5 Gew.-% Photoinitiator(en),
(c) 1 bis 40 Gew.-%, bevorzugt 5 bis 40 Gew.-% und besonders bevorzugt 10 bis 30 Gew.-% radikalisch polymerisierbare(s) Monomer(e),
(d) 30 bis 90 Gew.-%, bevorzugt 40 bis 90 Gew.-% und besonders bevorzugt 40 bis 85 Gew.-% Keramik- und/oder Glaskeramikpartikel als Füllstoff(e), und
(f) ggf. 0,001 bis 15 Gew.-%, bevorzugt 0,1 bis 15 Gew.-% und besonders bevorzugt 0,1 bis 13 Gew.-% Additiv(e).

Wenn nicht anders angegeben beziehen sich alle Mengenangaben hierin auf die Gesamtmasse der Materialien. Die einzelnen Mengenbereiche können separat gewählt werden.

Besonders bevorzugt sind solche Werkstoffe, welche aus den genannten Komponenten bestehen. Weiterhin sind solche Werkstoffe bevorzugt, bei denen die einzelnen Komponenten jeweils aus den oben genannten bevorzugten und besonders bevorzugten Stoffen ausgewählt sind. Besonders bevorzugt sind außerdem Werkstoffe, die ggf. neben der Verbindung der Formel (1) keine flüchtigen Mercaptane enthalten, d.h. Mercaptane, die einen typischen Mercaptangeruch aufweisen. Ganz besonders bevorzugt sind Zusammensetzungen, die keine weiteren Mercaptane und vorzugsweise auch keine anderen Schwefelverbindungen enthalten. Weiter bevorzugt sind Zusammensetzungen, die neben den Verbindungen der Formel 1 keine anderen Kettenregler enthalten.

Die erfindungsgemäßen Werkstoffe eignen sich besonders als Dentalwerkstoffe, insbesondere als dentale Zemente, Füllungskomposite und Verblendmaterialien sowie als Materialien zur Herstellung von Prothesen, künstlichen Zähnen, Inlays, Onlays, Kronen und Brücken. Sie haben ähnliche mechanische Eigenschaften (Biegefestigkeit und E-Modul) wie auf Dimethacrylaten basierende Materialien, zeichnen sich jedoch durch eine verringerte Polymerisationsschrumpfungsspannung (PKS), eine verbesserte Schlagzähigkeit und geringen Eigengeruch aus.

Die Dentalwerkstoffe eignen sich primär zur intraoralen Anwendung durch den Zahnarzt zur Restauration geschädigter Zähne (klinische Materialien), d.h. zur therapeutischen Anwendung, z.B. als dentale Zemente, Füllungskomposite und Verblendmaterialien. Sie können aber auch extraoral eingesetzt werden, beispielsweise bei der Herstellung oder Reparatur von Dentalrestaurationen, wie Prothesen, künstlichen Zähnen, Inlays, Onlays, Kronen und Brücken (technische Materialien).

Ein weiterer Gegenstand der Erfindung sind Homo- und Copolymerisate, die durch Polymerisation erfindungsgemäßer Dentalwerkstoffe erhalten werden könne. Derartige Polymerisate lassen sich beispielsweise durch spanabhebende Verfahren zu Prothesen oder künstlichen Zähnen verarbeiten. Sie liegen vorzugsweise in Form von zylinderförmigen oder scheibenförmigen Rohlingen vor.

Die erfindungsgemäßen Materialien eignen sich außerdem zur Herstellung von Formkörpern, welche z.B. mittels Gießen, Pressen oder 3D Druck hergestellt werden können. Besonders die verbesserte Schlagzähigkeit lässt diese Materialien nun auf gleiches Niveau wie gängigen Thermoplasten kommen. Zusätzlich ist die geringe Verzögerung bei der Härtung für den 3D Druck essentiell.
Außerdem betrifft die Erfindung die Verwendung von Verbindungen der Formel 1 wie in den Ansprüche definiert sind zur Verringerung der Polymerisationsschrumpfungsspannung und/oder zur Verbesserung der Schalgzähigkeit von Polymerisaten.

Die Erfindung wird im Folgenden anhand von Figuren und Ausführungsbeispielen näher erläutert.
Fig. 1 ist ein Photo eines Biegetests mit einem erfindungsgemäßen Polymerisat.
Fig. 2a und 2b zeigen jeweils ein Photo eines Schrumpfungstests.
Fig. 3, 4a und 4b zeigen jeweils ein Photo der Bruchfläche eines Polymerisats.

### Ausführungsbeispiele

### Beispiel 1:

### Synthese von 2-Methansulfonyloxyacrylsäureethylester (1)

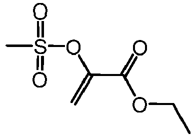

Eine Lösung von Brenztraubensäureethylester (23,22 g, 0,20 mol) in Dichlormethan (200 ml) wurde bei -5 °C mit Triethylamin (TEA, 24,23 g, 0,24 mol) versetzt. Methansulfonylchlorid (27,49 g, 0,24 mol) wurde zugetropft. Anschließend wurde das Reaktionsgemisch zunächst 1 h bei -5 °C und dann bei Umgebungstemperatur weitergerührt. Nach 22 h wurde die gelbe Reaktionslösung mit Wasser (5x 100 ml) und gesättigter wässriger NaCl-Lösung (100 ml) gewaschen, über wasserfreiem Na₂SO₄ getrocknet, filtriert und am Rotationsverdampfer eingeengt. Das Rohprodukt wurde mittels Säulenchromatographie (SiO₂, *n-*Hexan/Ethylacetat 9:1) gereinigt. Man erhielt 14,99 g (39 % Ausbeute) einer gelblichen Flüssigkeit.
¹H-NMR (CDCl₃, 400 MHz) : δ = 1,35 (t, 3H; *J* = 7,2 Hz; CH₃), 3,28 (s, 3H; S-CH₃), 4,31 (q, 2H; *J* = 7,2 Hz; O-CH₂), 5,81 (d, 1H; *J =* 2,3 Hz; =CH), 6,22 (d, 1H; *J =* 2,3 Hz; =CH).
¹³C-NMR (CDCl₃, 100,6 MHz): δ = 13,9 (CH₃), 38,8 (S-CH₃), 62,2 (O-CH₂), 117,4 (=CH₂), 142,9 (=C) , 161,0 (C=O).
IR (neat): 2986 (w), 2942 (w), 1731 (s), 1638 (m), 1468 (w), 1362 (s), 1334 (m), 1296 (s), 1187 (s), 1172 (m), 1136 (vs), 1019 (m), 956 (s), 895 (m), 861 (m), 790 (s), 684 (m), 627 (m) cm⁻¹.
Elementaranalyse: Ber. für C₆H₁₀O₅S: C: 37,11; H: 5,19; S : 16,51. Gef.: C: 37,31; H: 5,18; S: 16,40.

### Beispiel 2:

### Synthese von 2-(Toluol-4-sulfonyloxy)-acrylsäureethylester (2)

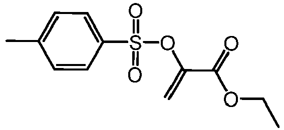

Zu einer Lösung von Brenztraubensäureethylester (5,81 g, 50,0 mmol), p-Toluolsulfonylchlorid (11,44 g, 60,0 mmol) und *N,N-*Dimethylaminopyridin (DMAP, 0,44 g, 3,6 mmol) in Dichlormethan (100 ml) wurde TEA (9,11 g, 90,0 mmol) zugetropft. Die Reaktionslösung wurde 24 h bei Umgebungstemperatur gerührt, mit Wasser (3x 100 ml) und gesättigter wässriger NaCl-Lösung (100 ml) gewaschen, über wasserfreiem Na₂SO₄ getrocknet, filtriert und am Rotationsverdampfer eingeengt. Das Rohprodukt wurde mittels Säulenchromatographie (SiO₂, n-Hexan/Ethylacetat 9:1) gereinigt. Man erhielt 8,88 g (66 % Ausbeute) eines gelblichen Öls.
¹H-NMR (CDCl₃, 400 MHz) : δ = 1,22 (t, 3H; *J* = 7,2 Hz; CH₃), 2,45 (s, 3H; Ar-CH₃), 4, 15 (q, 2H; *J* = 7,2 Hz; O-CH₂), 5,62 (d, 1H; *J* = 2, 1 Hz; =CH), 6, 14 (d, 1H; *J* = 2,4 Hz; =CH), 7,36 (d, 2H; *J =* 8,2 Hz; Ar-H), 7,83 (d, 2H; *J =* 8,4 Hz; Ar-H).
¹³C-NMR (CDCl₃, 100,6 MHz) : δ = 13,8 (CH₃), 21,5 (Ar-CH₃), 61,9 (O-CH₂), 116,8 (=CH₂), 128,4 (Ar-CH), 129,6 (Ar-CH), 132,4 (Ar-C), 143,2 (=C), 145,5 (Ar-C), 160,7 (C=O).
IR (neat): 2984 (w), 1732 (s), 1639 (m), 1597 (m), 1494 (w), 1447 (w), 1372 (s), 1293 (s), 1194 (s), 1178 (s), 1140 (vs), 1090 (s), 1018 (m), 955 (s), 894 (m), 860 (m), 815 (m), 781 (m), 710 (s), 695 (s), 660 (s) cm⁻¹.
Elementaranalyse: Ber. für C₁₂H₁₄O₅S: C: 53,32; H: 5,22; S: 11,86. Gef.: C: 53,49; H: 5,23; S: 11,57.

### Beispiel 3:

### Synthese von Triethylenglykol-bis[2-(toluol-4-sulfonyloxy)-acrylat] (3)

### 1.Stufe: Triethylenglykoldipyruvat

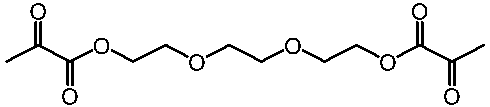

Eine Lösung von Brenztraubensäure (9,25 g, 0,105 mol), Triethylenglykol (7,51 g, 50,0 mmol) und DMAP (0,60 g, 5,0 mmol) in Dichlormethan (100 ml) wurde auf -5 °C abgekühlt. *N,N*'-Dicyclohexylcarbodiimid (22,69 g, 0,11 mol) wurde langsam zugegeben. Das Reaktionsgemisch wurde 2 h bei 0 °C und anschliessend bei RT weitergerührt. Nach 24 h wurde die Suspension über eine Schicht Kieselgel filtriert (SiO₂, Ethylacetat). Das Filtrat wurde am Rotationsverdampfer eingeengt. Das Rohprodukt wurde mittels Säulenchromatographie (SiO₂, *n-*Hexan/Ethylacetat 1:1) gereinigt. Man erhielt 6,08 g (42 % Ausbeute) einer gelben Flüssigkeit.
¹H-NMR (CDCl₃, 400 MHz): δ = 2,48 (s, 6H; CH₃), 3,67 (s, 4H; CH₂) , 3,80 (m, 4H; CH₂) , 4,40 (m, 4H; CH₂) .
¹³C-NMR (CDCl₃, 100,6 MHz): δ = 26,4 (CH₃), 65,0 (CH₂), 68,2 (CH₂), 70, 3 (CH₂), 160, 4 (C=O), 191, 3 (C=0) .
IR (neat) : 3452 (w), 2874 (w), 1727 (vs), 1450 (w), 1420 (w), 1358 (m), 1298 (s), 1123 (s), 1029 (m), 975 (m), 944 (m), 857 (m), 718 (m), 604 (m) cm⁻¹.
Elementaranalyse: Ber. für C₁₂H₁₈O₈: C: 49, 65; H: 6,25. Gef.: C: 49,16; H: 6,45.

### 2. Stufe: Triethylenglykol-bis[2-(toluol-4-sulfonyloxy)-acrylat] (3)

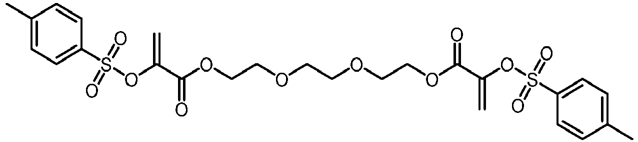

Triethylenglykoldipyruvat (5,81 g, 20,0 mmol), *p*-Toluolsulfonylchlorid (9,53 g, 50,0 mmol) und *N,N*-Dimethylaminopyridin (0,36 g, 3,0 mmol) wurden in Dichlormethan (100 ml) gelöst und Triethylamin (7,29 g, 72,0 mmol) wurde zugetropft. Das Reaktionsgemisch wurde 24 h bei RT gerührt, mit Wasser (3x 100 ml) und gesättigter wässriger NaCl-Lösung (100 ml) gewaschen, über Na₂SO₄ getrocknet, filtriert und am Rotationsverdampfer eingeengt. Das braune Öl wurde in n-Hexan/Ethylacetat 1:1 (25 ml) und Dichlormethan (5 ml) gelöst und über eine Schicht Kieselgel filtriert (SiO₂, *n*-Hexan/Ethylacetat 1:1). Das Filtrat wurde am Rotationsverdampfer eingeengt. Das bräunliche Öl wurde mit Diethylether (100 ml) versetzt und. Es bildete sich ein bräunlicher Niederschlag. Dieser wurde abfiltriert, durch wiederholtes Digerieren mit Diethylether weiter gereinigt und im Vakuumtrockenschrank getrocknet. Man erhielt 3,64 g (30 % Ausbeute) eines weissen Feststoffs (Smp.: 94 °C).
¹H-NMR (CDCl₃, 400 MHz) : δ = 2,45 (s, 6H; Ar-CH₃), 3,65 (s, 4H; CH₂), 3,68 (m, 4H; CH₂), 4,26 (m, 4H; CH₂), 5,60 (d, 2H; *J =* 2,2 Hz; =CH), 6, 15 (d, 2H; *J* = 2,2 Hz; =CH), 7,36 (d, 4H; *J =* 8,1 Hz; Ar-H), 7,84 (d, 4H; *J =* 8,2 Hz; Ar-H).
¹³C-NMR (CDCl₃, 100,6 MHz) : δ = 21,6 (CH₃), 64,9 (CH₂), 68,5 (CH₂), 70,5 (CH₂), 117,1 (=CH₂), 128,5 (Ar-CH), 129,7 (Ar-CH), 132,3 (Ar-C), 142,9 (=C), 145,6 (Ar-C), 160,8 (C=O).
IR (neat) : 3053 (w), 2958 (w), 2911 (w), 2865 (w), 1735 (s), 1636 (m), 1596 (m), 1495 (w), 1460 (m), 1372 (s), 1328 (w), 1301 (m), 1250 (w), 1192 (s), 1177 (s), 1147 (s), 1130 (vs), 1088 (s), 1051 (m), 1017 (m), 958 (s), 906 (w), 875 (m), 850 (m), 823 (m), 806 (m), 798 (m), 784 (s), 713 (s), 693 (s), 658 (s), 638 (m) cm⁻¹.
Elementaranalyse: Ber. für C₂₆H₃₀O₁₂S₂: C: 52,17; H: 5, 05; S: 10,71. Gef.: C: 51,93; H: 5,45; S: 10,70.

### Beispiel 4:

### Synthese von 2-(Toluol-4-sulfonylmethyl)-acrylsäureethylester (4) (Vergleichsbeispiel)

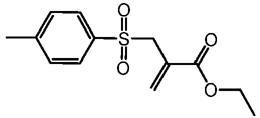

Ethyl-2-(bromomethyl)acrylat (1,13 g, 5,8 mmol), Natrium-p-toluolsulfinat (1,15 g, 6,4 mmol) und 0,12 g Polyethylenoxid 400 wurden unter Argon-Atmosphäre in 10 ml absolutem THF vorgelegt. Anschließend wurde für 5 Stunden auf Rückfluss erhitzt, wobei der Reaktionsfortschritt mittels NMR-Spektroskopie und Dünnschichtchromatographie überwacht wurde. Nach vollständiger Reaktion wurde die Reaktionslösung mit 10 ml deionisiertem Wasser und 10 ml Diethylether verdünnt. Die wässrige Phase wurde dreimal mit je 25 ml Diethylether extrahiert. Die vereinigten organischen Phasen wurden danach mit Brine gewaschen, über Natriumsulfat getrocknet und einrotiert. Das erhaltene Rohprodukt wurde säulenchromatographisch mit einem Gemisch aus PE/EE 2/1 gereinigt. (R_{f} ∼ 0,53). Ausbeute 86%.
¹H-NMR (200 MHz, CDCl₃, δ): 1,17 (t, *J* = 7,2 Hz, 3H; -COO-CH₂-CH₃), 2,43 (s, 3H; Ar-CH₃), 4,02 (q, *J* = 7,2 Hz, 2H; -COO-CH₂-CH₃), 4, 13 (s, 2H; -SO₂-CH₂-C-) , 5,89 (s, 1H; =CH₂), 6,49 (s, 1H; =CH₂), 7,32 (d, *J* = 8,2 Hz, 2H; Ar-H), 7,73 (d, *J =* 8,2 Hz, 2H; Ar-H).
¹³C-NMR (50 MHz, CDCl, δ) : 14, 0 (C1), 21,6 (C1), 57,5 (C2), 61,4 (C2), 128,8 (C3), 129,2 (C4), 129,6 (C3), 133,2 (C2), 135,4 (C4), 144.8 (C4), 164,8 (C=O).

### Beispiel 5:

### Synthese von Triethylenglykol-bis[2-methansulfonyloxy)acrylat] (5)

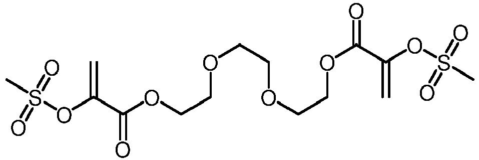

Eine Lösung von Triethylenglykoldipyruvat (5.19 g, 17.9 mmol) in Dichlormethan (100 ml) wurde bei -5 °C mit Triethylamin (15.69 g, 0.155 mol) versetzt. Methansulfonylchlorid (17.76 g, 0.155 mol) wurde zugetropft. Anschließend wurde das Reaktionsgemisch zunächst 1 h bei -5 °C und dann bei Umgebungstemperatur weitergerührt. Nach 24 h wurde die gelb-braune Reaktionslösung mit Wasser (5x 100 ml) und gesättigter wässriger NaCl-Lösung (100 ml) gewaschen, über Na₂SO₄ getrocknet, filtriert und am Rotationsverdampfer eingeengt. Das Rohprodukt wurde mittels Säulenchromatographie (SiO₂, n-Hexan/Aceton 3:2; R_{F} = 0.22) gereinigt. Man erhielt 1.69 g (3.8 mmol; 21%) einer gelblichen Flüssigkeit.
¹H-NMR (CDCl₃, 400 MHz) : δ = 3.28 (s, 6H; CH₃), 3.65 (s, 4H; CH₂), 3.75 (m, 4H; CH₂), 4.39 (m, 4H; CH₂), 5.83 (d, 2H; *J* = 2.4 Hz; =CH), 6.26 (d, 2H; *J* = 2.4 Hz; =CH).
¹³C-NMR (CDCl₃, 100.6 MHz) : δ = 38.9 (CH₃), 65.1 (CH₂), 68.7 (CH₂), 70.5 (CH₂), 118.2 (=CH₂), 142.7 (=C), 161.1 (C=0) .
IR (neat) : 3025 (w), 2940 (w), 1732 (s), 1638 (m), 1454 (w), 1358 (s), 1294 (s), 1185 (s), 1135 (vs), 1031 (m), 954 (vs), 863 (m), 789 (s), 686 (s), 628 (m) cm⁻¹.

### Beispiel 6:

### Synthese von Diethyl-2,2'-([1,3-phenyldisulfonyl]bis[oxy])-diacrylat 6

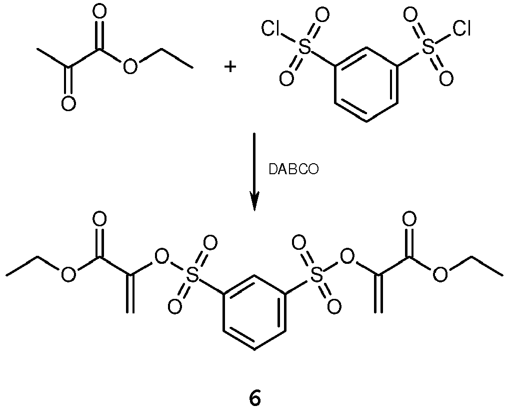

Zu einer Lösung von 1,4-Diazabicyclo[2.2.2]octan (13,05 g, 38,8 mmol) in abs. CH₂Cl₂ (50 ml) wurde unter Rühren und Argonatmosphäre eine Lösung von Ethylpyruvat (9,70 g, 83,5 mmol) und Benzoldisulfonylchlorid (10,66 g, 116,3 mmol) in abs. CH₂Cl₂ (150 ml) zugetropft. Die Reaktionslösung wurde 72 h lang bei Raumtemperatur gerührt, mit CH₂Cl₂ (200 ml) verdünnt und durch Kieselgel filtriert. Die Aufarbeitung erfolgte mittels Ausschütteln mit 1%iger HCl (2x 150 ml), entionisiertem Wasser (2x 150 ml) und gesättigter wässriger NaCl-Lösung (150 ml). Die organische Phase wurde über wasserfreiem Na₂SO₄ getrocknet, filtriert und am Rotationsverdampfer eingeengt, wobei 12,38 g (73 % d. Th.) von (**6**) als leicht viskose, gelbliche Flüssigkeit mit einer NMR-Reinheit von 98 % erhalten wurden. Rf: 0,50 (PE/EE 2:1)
¹H-NMR (CDCl₃, 200 MHz): δ = 8,46 (t, 1H, J = 1,47 Hz, ar-2H), 8,21 (t, 2H, J = 7,33 Hz, ar-4H, ar-6H) 7,77 (t, 1H, J = 7,93, ar-5H), 6, 16 (d, 2H, J = 2,49 Hz, 2x >C=CH₂, cis), 5,69 (d, 2H, J = 2,54 Hz, 2x >C=CH₂, trans), 4,09 (q, 4H, J = 6,79 Hz, 2x 0-CH₂-), 1,17 (t, 6H, J = 7, 14 Hz, 2x -CH₃).
¹³C-NMR (CDCl₃, 50 MHz, δ): 181,9 (>C=), 160,4 (O-C=O), 143, 0 (ar-S), 133,9 (ar-C4, ar-C6), 133,7 (ar-C5), 134,4 (ar-C2), 117,8 (=CH₂), 62,2 (O-CH₂-), 13,8 (-CH₃).

### Beispiel 7:

### Reaktivitätsmessungen

Um die Reaktivität der Verbindungen der Formel 1 mit bekannten Konzepten zu vergleichen, wurden mit den hergestellten Mischungen Photo-DSC-Messungen durchgeführt. Als unmodifiziertes Basis-Harz wurde ein äquimolares Gemisch der kommerziell erhältlichen Dimethacrylate Urethandimethacrylat (UDMA, Isomerengemisch; CAS: 72869-86-4) und 1,10-Decandioldimethacrylat (D₃MA) (Harzmischung 2M) eingesetzt, um vernetzte Polymerisate zu erhalten. Dieser Basismischung wurden anschließend 0,20 Doppelbindungsäquivalente des jeweiligen Reglers der Formel 1 zugesetzt. Bei Verbindungen mit ungenügender Löslichkeit wurde der Anteil auf 0,05 Doppelbindungsäquivalente verringert (in Tabelle 4 mit * gekennzeichnet).

Für die Polymerisation wurde ein Netzsch DSC 204 F1 mit Autosampler verwendet. Die Messung wurde isotherm bei 25°C unter Stickstoffatmosphäre durchgeführt. 10 ± 1 mg Probenmischung wurden in ein Aluminum-DSC-Schälchen eingewogen, das mittels Autosampler in der DSC Kammer platziert wurde. Die Probe wurde 4 min lang mit Stickstoff gespült (20 ml/min) und anschließend 5 min lang mittels gefiltertem UV-Licht (400-500 nm; Omnicure 2000) mit einer Intensität von 1 W/cm² am Strahlenausgang der Lampe bestrahlt. Zur Beurteilung der Reaktivität wurden die Zeit bis zum Erreichen von 95 % des maximalen Umsatzes (t₉₅) und die Zeit bis zum Erreichen der maximalen Polymerisationsgeschwindigkeit (tₘₐₓ) herangezogen.

Da neben der Polymerisationsgeschwindigkeit auch die Regelung des Molekulargewichts essentiell ist, wurden zusätzlich Proben mit monofunktionellem Benzylmethacrylat (BMA) hergestellt. Die Proben wurden ebenfalls 4 min lang mit Stickstoff gespült (20 ml/Min) und anschließend 5 min lang mittels gefiltertem UV-Licht (400-500 nm; Omnicure 2000) mit einer Intensität von 1 W/cm² am Strahlenausgang der Lampe bestrahlt. Die polymerisierten Proben wurden in THF gelöst und mit einer Waters GPC mit drei in Serie geschalteten Säulen (Styragel HR 0.5, Styragel HR 3 und Styragel HR 4) und einem Waters 2410 RI Detektor in einem Säulenofen bei 40°C und mit einer Flussrate von 1,0 ml/min analysiert. Zur Kalibrierung wurden Polystyrolstandards verwendet. Das Verhältnis zwischen dem zahlenmittleren Molekulargewicht des modifizierten Polymers und jenem von reinem Poly-BMA (Mn_{mod/}Mn_{BMA}) zeigt, wie stark das durchschnittliche Molekulargewicht durch den Regler gesenkt wird. Wünschenswert ist eine starke Verringerung des Molekulargewichts, d.h. ein niedriger Wert des Verhältnisses Mn_{mod}/Mn_{BMA}, bei gleichzeitig hoher Polymerisationsgeschwindigkeit, d.h. relativ niedrigen Werten für t₉₅ und tₘₐₓ in der obigen Reaktion mit UDMA/D₃MA. Zusätzlich ist das Erreichen eines hohen Doppelbindungsumsatzes (DBC) erstrebenswert, um die nötigen mechanischen Eigenschaften des Polymerisats zu gewährleisten. In der Folge wird der Doppelbindungsumsatz für das Monomerengemisch UDMA/D₃MA als DBC_{U/D} und jener für das BMA-Monomer alleine mit DBC_{BMA} bezeichnet.

Als Regler wurden dabei die Verbindungen **1** und **2** gemäß vorliegender Erfindung mit anderen Verbindungen verglichen.

Bei Vergleichsbeispiel V1 handelte es sich entweder um das reine Methacrylat-basierte Gemisch aus gleichen Gewichtsteilen an UDMA und D₃MA, oder um das monofunktionelle Methacrylat BMA. Das Vergleichsbeispiel V2 setzt sich aus einer Mischung von V1 und dem β-Allylsulfon **4** (Beispiel 4), das als Analogon zur Verbindung **2** eingesetzt wurde, zusammen. V3 bis V12 sind Vergleichsbeispiele aus Methacrylaten (entweder auf UDMA/D3MA- oder BMA-Basis) mit unterschiedlichsten bekannten Reglern, die sich anhand ihrer Abgangsgruppe (Sulfon, Sulfid, Phosphon, Alkyl) bzw. Aktivierungsgruppe (Ester, Amid, aromatisch) unterscheiden. Bei V13 handelt es sich um eine Formulierung mit einem Bartonester als Regler.

**Tabelle 1**

| **Regler** | **Bsp.** | **t₉₅ [s]** | **tₘₐₓ [s]** | **Mn_{mod}/Mn_{BMA} [ ]** | **DBC_{U/D} // DBC_{BMA} [%] // [%]** |
|---|---|---|---|---|---|
| keiner (UDMA/D₃MA bzw. BMA) | V1 | 66,4 | 4,3 | - | 74 // - |
| 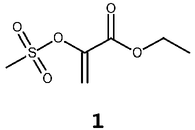 | 1 | 65,6 | 15,3 | 0,20 | 94 // 55 |
| 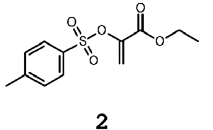 | 2 | 55,6 | 12,1 | 0,12 | 93 // 57 |
| 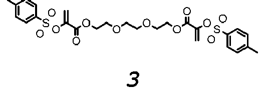 | 3 | 65,0 | 7,5 | 0,32 | 95 // 56 |
| 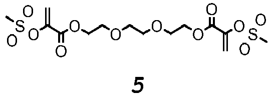 | 5 | 65,0 | 9,2 | 0,30 | 97 // 55 |

| **Regler** | **Bsp.** | **t₉₅ [s]** | **tₘₐₓ [s]** | **Mn_{mod}/Mn_{BMA} [ ]** | **DBC_{U/D} // DBC_{BMA}[%] // [%]** |
|---|---|---|---|---|---|
| 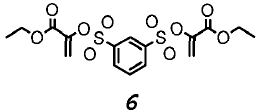 | 6 | 44,3 | 7,8 | 0,22 | 93 // 82 |
| 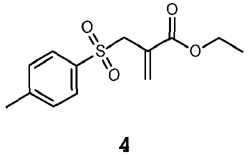 | V2 | 82,6 | 10,2 | 0,18 | 61 // 46 |
| 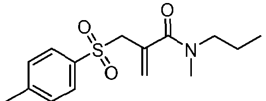 | V3 | 33,2 | 5,1 | 1,16 | 86 // 76 |
| 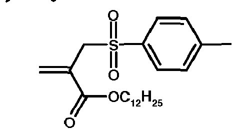 | V4 | 114,3 | 4,1 | 0,20 | 70 // 52 |
| 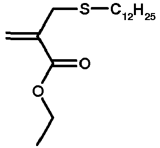 | V5 | 108 | 10,7 | 0,22 | 63 // 36 |
| 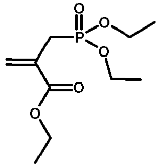 | V6 | 181,2 | 12 | 0,53 | 49 // 17 |
| 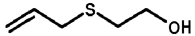 | V7 | 73 | 16, 1 | 0,47 | - // 21 |
| 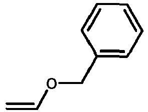 | V8 | 73,2 | 8,1 | 0, 66 | - // 16 |
| 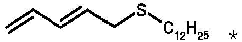 | V9 | 117,8 | 12, 3 | 0,40 | - // 13 |
| 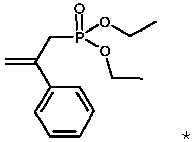 | V10 | 107,8 | 9,9 | 0,49 | - // 15 |

| **Regler** | **Bsp.** | **t₉₅ [s]** | **tₘₐₓ [s]** | **Mn_{mod}/Mn_{BMA} [ ]** | **DBC_{U/D} // DBC_{BMA} [%] // [%]** |
|---|---|---|---|---|---|
| 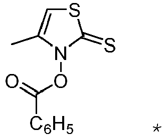 | V11 | 74,5 | 11 | 0,45 | 75 // 46 |
| 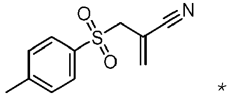 | V12 | 102,1 | 7,8 | 0,51 | 57 // 33 |
| 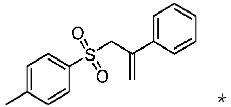 | V13 | 74 | 11, 3 | 0,43 | 64 // 26 |

| | | | | | |
|---|---|---|---|---|---|
| * geringe Löslichkeit, daher c = 5 DB% V Vergleichsbeispiel | | | | | |

Aus obiger Tabelle 1 geht klar hervor, dass die Verbindungen **1, 2, 3, 5** und **6** bei erfindungsgemäßer Verwendung als Regler überaschenderweise in der Lage waren, die Zeit t₉₅ bis zum Erreichen von 95 % Umsatz zu verkürzen, was von den Vergleichsbeispielen nur der Verbindung aus Vergleichsbeispiel V3, einem Allylsulfon, gelang. Allerdings erhöhte diese Verbindung das mittlere Molekulargewicht des erhaltenen Polymerisats sogar um 16 %, anstatt die Kettenlänge zu verringern. Im Gegensatz dazu bewirkten die Verbindungen gemäß vorliegender Erfindung eine deutliche Verringerung auf bis zu etwa 1/8 des nicht modifizierten Produkts. Gleichzeitig bewirkten sie jedoch eine Verlängerung der Zeit tₘₐₓ bis zum Erreichen der maximalen Polymerisationsgeschwindigkeit, mitunter auf das 3- bis 4fache (Verbindungen 1 und 2), was vorteilhaft ist, da es so erst viel später zum Gelieren des Reaktionsgemischs kommt, so dass die Polymerisation länger ungehindert unter im Wesentlichen homogenen Bedingungen ablaufen kann. Eine solche wünschenswerte Kombination von Eigenschaften zeigte kein einziges der Vergleichsbeispiele. Da die aus Moad et al. (s.o.) bekannte Verbindung **4** in Vergleichsbeispiel V2 diesbezüglich am besten abschnitt, wurde diese in der Folge für weitere Vergleiche mit den Sulfonsäureestern zur erfindungsgemäßen Verwendung herangezogen.

### Beispiel 8:

### Herstellung und Charakterisierung von Photopolymeren mit Dimethacrylaten und den Übertragungsreagenzien (Reglern) aus den Beispielen 1, 2 und 4

Es wurden Harzformulierungen aus einer 1/1-Mischung (mol/mol) von UDMA und D₃MA (Harzmischung 2M) und aus einer Mischung von UDMA, D₃MA mit Übertragungsreagenz **1, 2** bzw. **4** hergestellt (1/1/1-molare Mischung in Gew.-% Übertragungsreagenz angegeben). Alle Formulierungen enthielten zusätzlich ∼1 Gew.-% MBDEGe als Photoinitiator. Zur Überprüfung der Photoreaktivität wurden die hergestellten Formulierungen mit einem Photorheometer MCR302 WESP von Anton Paar vermessen, welches zur Umsatzkontrolle mit einem Bruker Vertex 80 IR-Spektrometer gekoppelt wurde. Es wurde ein PP-25 Messsystem verwendet, und der Messspalt wurde auf 0,1 mm eingestellt. Vor und während der Aushärtung (1 W/cm²; 400-500 nm; Omniucure 2000) wurden Speicher- und Verlustmodul der Probe im Osziallationsmodus (1% Auslenkung, 10 Hz) gemessen. Gleichzeitig wurden während der Messung IR Spektren der Probe mit einer Frequenz von -5 Hz aufgezeichnet. Als Maß der Photoreaktivität wurde das Erreichen des Gelpunktes (Schnittpunkt von Speicher- und Verlustmodul) und die Dauer bis zu 95% des Gesamtumsatzes (t_{95%}) herangezogen. Zusätzlich konnte der Umsatz am Gelpunkt (DBC_{g}), der Gesamtumsatz (DBC) und die Photopolymerisations induzierte Schrumpfungsspannung (Fₛ bzw. Fₛ bei 75% Umsatz F_{s(75%)}) ermittelt werden. Die erhaltenen Ergebnisse sind in Tabelle 2 zusammengefasst.

**Tabelle 2: RT-NIR-Photorheometrie**

| **Formulierung** | **Gelpunkt [s]** | **DBC_{g} [%]** | **DBC [%]** | **t_{95%} [S]** | **F_{S (75%)} [N]** | **F_{S} [N]** |
|---|---|---|---|---|---|---|
| 2M^{a)}* | 2,1 | 41 | 79 | 69, 0 | -14, 9 | -17, 9 |
| 2M + 20Gew.-% **1** | 10,4 | 46 | 95 | 48,2 | -9,9 | -19, 5 |
| 2M + 26 Gew.-% **2** | 7,9 | 56 | 93 | 25,9 | -8,8 | -15,6 |
| 2M + 26 Gew.-% **4*** | 12,4 | 38 | 76 | 110,5 | -9,7 | -12, 0 |

| | | | | | | |
|---|---|---|---|---|---|---|
| ^{a)} 2M: UDMA/D₃MA 1/1 * Vergleichsbeispiel | | | | | | |

Es ist klar ersichtlich, dass durch die Übertragungsreagenzien **1** und **2** und das Vergleichsreagenz **4** der Umsatz am Gelpunkt erhöht werden kann, was einen erhöhten Gesamtumsatz und eine reduzierte Schrumpfungsspannung bewirkt. Im Gegensatz zum Vergleichsreagenz **4,** wird mit den Übertragungsreagenzien **1** und **2** die Reaktionsdauer (t_{95%}) allerdings kaum beeinträchtigt und ein erheblich größerer Gesamtumsatz erzielt.

### Beispiel 9:

### DMTA Analyse der hergestellten Photopolymere

Zur Ermittlung des Glasüberganges wurden die in Beispiel 8 hergestellten Formulierungen in Silikonformen gegossen und in einem Lichtofen (Modell Lumamat 100, Ivoclar AG) mit dem Programm 2 (P2: 10 min Bestrahlung mit einer Intensität von ca. 20 mW/cm²) polymerisiert. Die Stäbe wurden gewendet und abermals mit P2 ausgehärtet. Die Probestäbchen wurden geschliffen und dann auf einem AntonPaar Rheometer MCR301 mit einem CTD-Ofen (Convection Temperature Control) und einer eingesetzten Festkörpereinspannvorrichtung (SRF12 für rechteckige Querschnitte bis 12 mm) vermessen. Die eingestellte Heizrate betrug 2 °C/min. Alle Proben wurden von -100 °C auf 200 °C aufgeheizt und mit einer konstanten Frequenz von 1 Hz und 0,1 % Auslenkung oszilliert. Die in Tabelle 3 gezeigten Messwerte zeigen, dass durch Zugabe der Übertragungsreagenzien **1, 2** und **4** ein niedrigerer und deutlich schmalerer Glasübergangsbereich erreicht werden kann.

**Tabelle 3: DMTA**

| **Formulierung** | **G'_{(20°)} [MPa]** | **T_{G} [°C]** | **HWB [°C]** | **G'ᵣ^{a)} [MPa]** |
|---|---|---|---|---|
| 2M^{b)}*) | 940 | 150 | 146 | 81,9 |
| 2M + 20 Gew.-% **1** | 999 | 69 | 23 | 6, 0 |
| 2M + 26 Gew.-% **2** | 1020 | 68 | 25 | 9, 8 |
| 2M + 26 Gew.-% **4***⁾ | 810 | 72 | 23 | 4,4 |

| | | | | |
|---|---|---|---|---|
| ^{a)} Speichermodul am Gummielastischen-Plateau ^{b)} 2M: UDMA/D₃MA 1/1 *⁾ Vergleichsbeispiel | | | | |

Durch Einsatz der Übertragungsreagenzien **1, 2** und **4,** kann die Glasübergangstemperatur (T_{G}) eingestellt und ein scharfer Glasübergangsbereich erhalten werden (charakterisiert über die Halbwertsbreite der Verlustfaktorkurve beim Glasübergang, HWB). Der Speichermodul bei Raumtemperatur (G'_{(20°C)}) bleibt nahezu unverändert und wird zum Teil sogar leicht verbessert. Der Effekt der Verbindungen 1, 2 und 4 ist vergleichbar. Die erfindungsgemäßen Verbindungen 1 und 2 bewirken aber anders als Verbindung 4 keine Verzögerung der Reaktion und ergeben einen höheren Umsatz (s. Bsp. 8).

### Beispiel 10:

### Messung der Schlagzähigkeit (Dynstat-Impact-Test)

Die Ermittlung der Zähigkeitseigenschaften erfolgte mittels der DYNSTAT-Anordnung entsprechend der DIN 53435, wobei die Schlagzähigkeit (Schlagarbeit) von ungekerbten Prüfkörpern in der Schlagbiegeanordnung ermittelt wurde. Es wurden Probestäbe (∼ 1 x 0,45 x 0,15 cm) mit verschiedenen Formulierungen hergestellt und Dynstat-Impact Tests (Verwendung eines 5 kpcm - Hammers; 0,5 J) durchgeführt. In der unten angeführten Tabelle 4 sind die erhaltenen Werte aufgelistet.

**Tabelle 4: Schlagzähigkeit**

| **Formulierung** | **Schlagarbeit [kJ/m²]^{a)}** |
|---|---|
| 2M^{b)}* | 2,4±0,4 |
| 2M + 20 Gew.-% **1** | 11,3±1,7 |
| 2M + 26 Gew.-% **2** | 17,4±1,1 |
| 2M + 26 Gew.-% **4*** | 6,1±1,75 |

| | |
|---|---|
| ^{a)} Normiert auf Breite und Dicke ^{b)} 2M: UDMA/D₃MA 1/1 * Vergleichsbeispiel | |

Es ist ersichtlich, dass mit den Verbidungen 1, 2 und 4 eine Schlagzähigkeitserhöhung erreicht werden konnte, wobei mit den Verbindungen **1** und **2** Zunahmen auf das 4,7- bzw. 7,25fache beobachtet wurden, während Vergleichsverbindung **4** nur eine Steigerung auf das 2,5fache bewirkte.

### Beispiel 11:

### Messung der Schlagzähigkeit (Dynstat-Impact-Test)

Die Ermittlung der Zähigkeitseigenschaften erfolgte analog zu den Beispiel 10. In der nachstehenden Tabelle 5 sind die erhaltenen Werte für Formulierungen mit GDVA-Monomer alleine sowie mit 24 Gew.-% der Verbindung **2** aufgelistet.

**Tabelle 5**

| **Formulierung** | **Schlagarbeit [kJ/m²]^{a)}** |
|---|---|
| GDVA*) | 5,1 ± 1,0 |
| GDVA + **2** | - |

| | |
|---|---|
| *) Vergleichsbeispiel ^{a)} Normiert auf Breite und Dicke | |

Nur die Probenkörper aus reinem GDVA konnten mit dieser Prüfanordnung zerbrochen werden, während die gemäß vorliegender Erfindung hergestellten Prüfkörper mit 24 Gew.-% der Verbindung **2** so hohe Zähigkeit aufwiesen, dass sie den Schlagbiegeversuch unbeschadet überstanden.

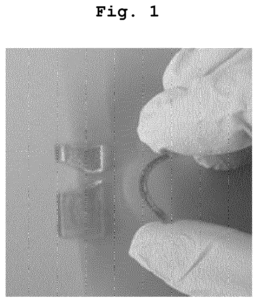

Zur Interpretation dieses Ergebnisses wurden die beiden erhaltenen Photopolymere händisch gebogen. Homopolymere aus GDVA waren sehr spröde und brachen relativ leicht, erfindungsgemäße Polymerisate mit Verbindung **2** ließen sich hingegen verbiegen, ohne zu brechen, wie dies in Fig. 1 für ein Polymerisat aus GDVA (links) und eines aus GDVA und Verbindung **2** dargestellt ist.

### Beispiel 12:

### Herstellung und Charakterisierung von Gelen mit Polyethylenglykoldiacrylat

Es wurden Formulierungen mit Polyethylenglykoldiacrylat (PEGDA, Mw ∼ 750 g/mol) alleine und mit jeweils 19 Gew.-% der Verbindungen **2** bzw. **4** hergestellt. Alle Formulierungen enthielten zusätzlich ∼ 0,5 Gew.-% Ivocerin als Photoinitiator. Die hergestellten Harzformulierungen wurden mit 60 Gew.-% Dimethylsulfoxid (DMSO) vermischt, und zur Überprüfung der Photoreaktivität wurden diese Formulierungen analog zu Beispiel 8 mit dem Photorheometer MCR302 WESP von Anton Paar vermessen, das zur Umsatzkontrolle mit einem Bruker Vertex-80-IR-Spektrometer gekoppelt wurde. Die dabei erzielten Ergebnisse sind in Tabelle 6 angeführt.

**Tabelle 6: RT-NIR-Photorheometrie**

| **Formulierung** | **Gelpunkt [s]** | **DBC_{g} [%]** | **DBC [%]** | **F_{S} [N]** |
|---|---|---|---|---|
| PEGDA* ) | 66 | >99 | >99 | -6,6 |
| PEGDA + **2** | 70 | 88 | >99 | -4,5 |
| PEGDA + **4***) | 205 | 41 | 48 | -0,2 |

| | | | | |
|---|---|---|---|---|
| *) Vergleichsbeispiel | | | | |

Ähnlich wie bei der Photopolymerisation von Di(meth)acrylaten ohne DMSO kommt es auch bei der Homopolymerisation von PEGDA zu einer rapiden Gelbildung, was bei ungeregelter Reaktion zu hoher Schrumpfungsspannung (-6,6 N), allerdings auch nahezu quantitativen Umsätzen (> 99%) führt. Es ist klar ersichtlich, dass durch Verbindung **4** auch die Photopolymerisation von Acrylat-basierten DMSO-Gelen inhibiert wird. Der Gelpunkt wird mit >200 s erst sehr spät erreicht, und der Umsatz ist mit unter 50 % sehr gering, was auch die sehr geringe Schrumpfspannung erklärt. Mit Verbindung **2** kann gemäß vorliegender Erfindung die PEGDA-Polymerisation in DMSO gut geregelt werden, und es kommt zu einer stark reduzierten Schrumpfungsspannung bei gleichbleibendem hohem Umsatz.

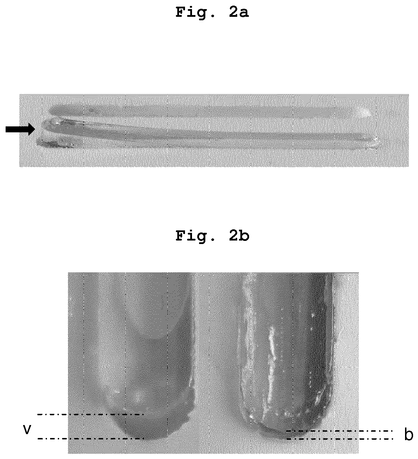

Die gemessene reduzierte Schrumpfungsspannung nach Zusatz von Verbindung **2** wurde auf folgende Weise illustriert: Es wurde eine optische Analyse des Polymerisationsschrumpfes durchgeführt, wozu die DMSO-basierten PEGDA-Formulierungen mit Verbindung **2** als Regler in eine Teflonform gegossen und im Lumamat 100 für 10 min bestrahlt wurden. Fig. 2 zeigt die so erhaltenen Gele.

In den beiden Photos in Fig. 2 ist zu erkennen, dass sich das ohne Regler hergestellte Gel krümmt (Fig. 2a, unten; durch den Pfeil angezeigt) und das mit Verbindung **4** hergestellte Gel seine Länge gegenüber jenem aus dem Vergleichsbeispiel ohne Regler stark verkürzt (Fig. 2b, links; Längendifferenz "v"). Das erfindungsgemäß mit Verbindung 2 als Regler hergestellte Gel (Fig. 2a, oben; Fig. 2b, rechts) schrumpfte aufgrund der reduzierten Schrumpfungsspannung während der Polymerisation hingegen kaum (Längendifferenz "b").

### Beispiel 13:

### Herstellung und Charakterisierung von Beschichtungen mit Urethandiacrylat

Es wurden Formulierungen mit dem Urethandiacrylat (UDA) Ebecryl 2002 von Sartomer alleine und mit jeweils 20 Gew.-% der Verbindungen **2** bzw. **4** hergestellt. Alle Formulierungen enthielten zusätzlich ∼ 1 Gew.-% Darocur 1173 (BASF) als Photoinitiator. Zur Überprüfung der Photoreaktivität wurden diese Formulierungen analog zu Beispiel 8 mit dem Photorheometer MCR302 WESP von Anton Paar vermessen, das zur Umsatzkontrolle mit einem Bruker Vertex-80-IR-Spektrometer gekoppelt wurde. Die dabei erzielten Ergebnisse sind in Tabelle 7 angeführt.

**Tabelle 7: RT-NIR-Photorheometrie**

| **Formulierung** | **t₉₅ [s]** | **DBC [%]** | **F_{S} [N]** |
|---|---|---|---|
| UDA*)*) | 69 | 96 | -7,6 |
| UDA + **2** | 116 | 99 | -0,2 |
| UDA + **4***) | 185 | 73 | -5,2 |

| | | | |
|---|---|---|---|
| *) Vergleichsbeispiel | | | |

Man erkennt aus Tabelle 7, dass das erfindungsgemäße Polymerisat gegenüber dem ohne Regler hergestellten Produkt den ohnehin hohen Umsatz sogar noch weiter steigern konnte, vor allem aber nahezu keinen Schrumpf aufwies. Mit Verbindung **4** als Regler war der Umsatz deutlich geringer als ohne Regler, und die Schrumpfung konnte nur wenig gesenkt werden. Zudem erhöhte Verbindung **4** im Vergleich zu Verbindung **2** im erfindungsgemäßen Beispiel die Reaktionsdauer beträchtlich, d.h. um das 2,7fache der Polymerisation ohne Regler, während sie in Beispiel 15 nur auf das 1,7fache anstieg.

### Beispiele 14:

### Gitterschnitttest von Beschichtungen nach DIN EN ISO 2409

Eine anodisierte Aluminiumfolie wurde analog zu den obigen Beispielen mit UDA als Monomer bzw. den hergestellten Formulierungen mit 10 oder 20 Gew.-% der Verbindungen **2** bzw. **4** beschichtet (4-mil-Rakel, ∼ 102 µm) und in einem UV-Ofen ausgehärtet. Die Beschichtungen wurden mittels Gitterschnittprüfer (6 x 2 mm) geschnitten und dann gleichmäßig Klebeband (Tesafilm Standard 19 mm) über die geschnittenen Beschichtungen geklebt. Die Klebebänder wurden gleichmäßig in einem Winkel von ∼ 60° abgezogen, und das Erscheinungsbild der zurückbleibenden Gitter wurde beurteilt. Tabelle 8 zeigt die dabei erzielten Ergebnisse.

**Tabelle 8: Gitterschnittest**

| **Formulierung** | **Kennwert ISO** |
|---|---|
| UDA | GT 3 |
| UDA + 10 Gew.-% **2** | GT 1 |
| UDA + 20 Gew.-% **2** | GT 1 |
| UDA + 10 Gew.-% **4***) | GT 1 |
| UDA + 20 Gew.-% **4***) | GT 1 |

| | |
|---|---|
| *) Vergleichsbeispiel | |

Die UDA-basierte Beschichtung ohne Regler wies etliche abgeplatzte Quadrate auf, wohingegen bei allen mit Regler hergestellten Beschichtungen - sowohl mit 10 Gew.-% als auch mit 20 Gew.-% Regler - nur kleine Splitter der Beschichtung an den Schnittpunkten und Schnitträndern der Gitterlinien abgeplatzt waren. Die Wirkung der Verbindungen **2** und **4** als Regler war in dieser Hinsicht gleichwertig.

### Beispiel 15:

### 3D-Druck

Für den 3D-Druck wurden Reaktionsgemische auf Basis eines aliphatischen Polyesterurethanmethacrylats (Bomar XR 741) mit 1 Gew.-% Ivocerin® als Photoinitiator hergestellt. Als Regler wurden 5, 7 bzw. 10 Gew.-% von Verbindung **2** zugemischt. Gemäß einer Literaturvorschrift (Liska et al., J. Polym. Sci. A Polym. Chem. 49, 4927-4934 (2011)) wurden dann Probestäbe vom Typ 5B nach DIN EN ISO 527-2 gedruckt und Zugversuche auf einer Zwick-500-Zugprüfmaschine durchgeführt. Die in Tabelle 9 angegebenen Ergebnisse stellen jeweils Mittelwerte von zumindest 6 Messungen dar.

**Tabelle 9: Bruchdehnung von 3D-Drucken**

| **Formulierung** | **Bruchdehnung [%]** |
|---|---|
| Reaktionsgemisch ohne Regler*) | 5,01 |
| Reaktionsgemisch + 5 % Gew.-% **2** | 7,12 |
| Reaktionsgemisch + 7 % Gew.-% **2** | 8,78 |
| Reaktionsgemisch + 9 % Gew.-% **2** | 12,32 |

| | |
|---|---|
| *) Vergleichsbeispiel | |

Es ist klar zu erkennen, dass sich die Bruchdehnung durch erfindungsgemäßen Zusatz von Regler **2** verbesserte.

### Beispiel 16:

### Oberflächenuntersuchungen

Es wurden Monomerengemische (Harzmischung 2M) analog zu Beispiel 8 ohne Regler sowie mit 20 Gew.-% von Verbindung **1** oder 26 Gew.-% von Verbindung **2** hergestellt. Zur Ermittlung des Gefügebildes wurden die hergestellten Formulierungen in Silikonformen gegossen und in einem Lichtofen (Modell Lumamat 100, Ivoclar AG) mit dem Programm 2 (P2: 10 min Bestrahlung mit einer Intensität von ca. 20 mW/cm²) polymerisiert. Die Stäbe (∼1 x 0,45 x 0,15 cm) wurden gewendet und abermals gemäß P2 ausgehärtet. Die Probestäbchen wurden geschliffen und dann mittels einer DYNSTAT-Anordnung entsprechend der DIN 53435, wobei die Prüfkörper ungekerbt waren, unter Verwendung eines 5-kpcm-Hammers analog zu Beispiel 10 geprüft. Die Bruchflächen wurden anschließend mittels REM auf einem SEM XL-30 von Philips analysiert. Dafür wurden die Proben auf einem Probenhalter mit Tape fixiert und die Ränder mit einer leitenden Silberlösung bestrichen. Anschließend wurden die Proben mit einer dünnen leitenden Goldschicht besputtert. Es wurden Bilder der Bruchflächen in 500facher Vergrößerung aufgenommen.

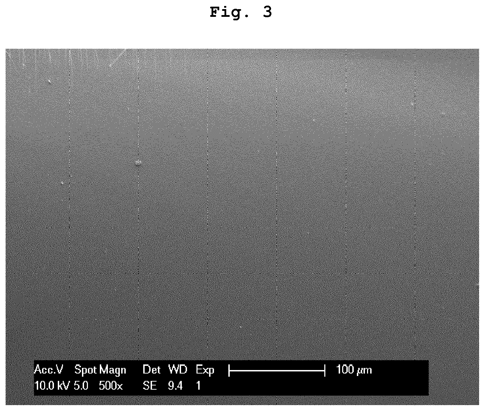

Fig. 3 zeigt die Bruchfläche des Polymerisats, das ohne Reglerzusatz hergestellt worden war: eine äußerst glatte Bruchfläche, die somit einen spröden Bruch anzeigt.

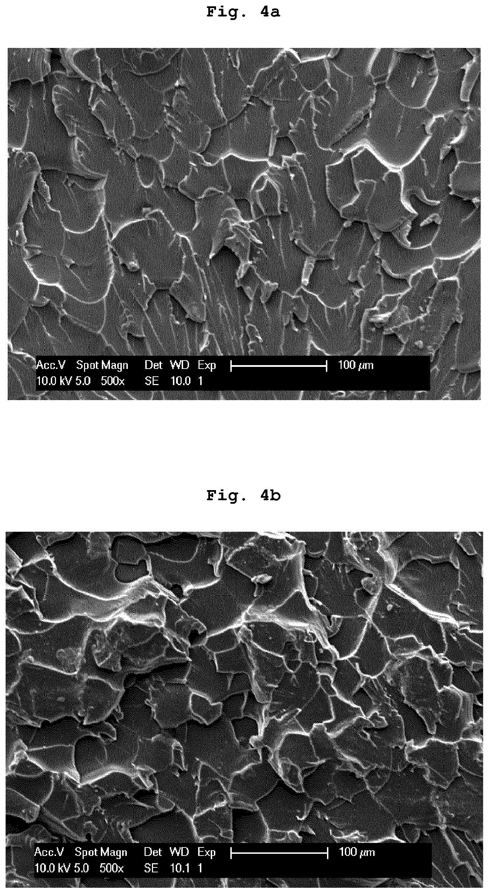

In Fig. 4 sind die Bruchflächen der erfindungsgemäßen Polymerisate mit 20 Gew.-% (Fig. 4a) bzw. 26 Gew.-% der Verbidnung **2** (Fig. 4b) zu sehen, die wesentlich duktiler sind als das ungeregelte Dimethacrylatnetzwerk.

### Beispiel 17:

### Molekulargewichtsbestimmung

Es wurden Polymerisate analog zu Beispiel 8 mit Benzylmethacrylat (BMA) ohne Regler sowie mit 22 Gew.-% von Verbindung **1,** 28 Gew.-% von Verbindung 2 und 28 Gew.-% von Verbindung **4** hergestellt, in THF gelöst und mit einer Waters GPC mit drei in Serie geschalteten Säulen (Styragel HR 0.5, Styragel HR 3 und Styragel HR 4) und einem Waters 2410 RI-Detektor in einem Säulenofen bei 40 °C und mit einer Flussrate von 1,0 ml/min unter Verwendung von Polystyrolstandards analysiert, wobei das Molekulargewicht (in kDa) und der Polydispersitätsindex (PDI) bestimmt wurden. Die Ergebnisse sind in Tabelle 10 angeführt.

**Tabelle 10: Molekulargewichtsbestimmung**

| **Beispiel** | **Mn [kDa]** | **PDI [ ]** |
|---|---|---|
| BMA*) | 7, 0 | 2,2 |
| BMA + **1** | 1,4 | 1,3 |
| BMA + **2** | 1,1 | 1,3 |
| BMA + **4***) | 1,3 | 1,3 |

| | | |
|---|---|---|
| *) Vergleichsbeispiel | | |

Die erhaltenen Werte, die gut mit jenen aus Tabelle 1 korrelieren, zeigen, dass die Polymerisation unter Zusatz der drei Regler zu deutlich kürzeren Ketten als bei der ungeregelten Reaktion führte. Darüber hinaus erwies sich erwartungsgemäß auch die Molekulargewichtsverteilung nach Reglerzusatz als erheblich gleichmäßiger. Verbindung **2** erwies sich in diesem Beispiel als wirksamster der drei getesteten Regler.

### Beispiel 18:

### Formulierung für den 3D-Druck

| **Komponente** | **Art** | **Menge (Gewichtsteile)** |
|---|---|---|
| Monomer | Dimethacrylate, z.B. Polyesterurethandimethacrylat Bomar XR 741 | 100 |
| Regler | Verbindung **6** | 12 |
| Initiator | Photoinitiator, etwa eine Bisacylgermanium-Verbindung, z.B. Ivocerin | 1 |
| Additive | UV-Absorber, z.B. Sudangelb | 0,2 |

Durch die Verwendung von Urethanmethacrylaten wird eine besonders gute Lagerstabilität erreicht. Die langwellige Absorption des Photoinitiators ist an das Emmissionsspektrum des 3D-Druckers angepasst. Der UV-Absorber verhindert Streulicht und kontrolliert die Schichtdicke der Härtung. Durch den erfindungsgemäßen Einsatz des Reglers kann die Schlagzähigkeit des Materials signifikant verbessert werden.

### Beispiel 19:

### Formulierung für Gießharze

| **Komponente** | **Art** | **Menge (Gewichtsteile)** |
|---|---|---|
| Monomere | Methacrylate, z.B. Methylmethacrylat | 75 |
| Regler | Formel 1 oder 2, n = 1, z.B. Verbindung **2** | 20 |
| Füllstoffe | Polymethylmethacrylat (MG 4, 76 x 10⁶) | 4 |
| Initiator | thermischer Initiator, z.B. AIBN | 1 |
| Additive | Formtrennmittel: Stearinsäure | 0,005 |

Durch die Verwendung von Methacrylaten werden besonders lichtstabile, thermisch härtbare Gießmassen erhalten. Der Einsatz relativ großer Mengen des Reglers verschiebt den Gelpunkt hin zu hohen Umsätzen, wodurch die Eigenspannungen im Material erheblich reduziert werden können.

## Patentansprüche

1. Radikalisch polymerisierbarer Dentalwerkstoff, der mindestens ein multifunktionelles (Meth)acrylat oder eine Mischung von mono- und multifunktionellen (Meth)acrylaten, mindestens einen Initiator für die radikalische Polymerisation und mindestens einen Sulfonsäureester der Formel 1 enthält: 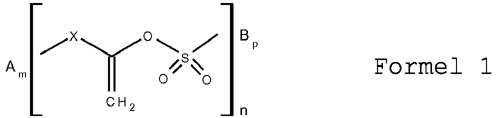 in der die Variablen die folgenden Bedeutungen haben:
A H, CN, ein cycloaliphatischer, linearer oder verzweigter aliphatischer C₁-C₃₀-Kohlenwasserstoffrest, der durch einen oder mehrere Substituenten substituiert sein kann, der durch eine oder mehrere Urethangruppen, Estergruppen, O und/oder S unterbrochen sein kann und der 1 bis 4 Benzolgruppen enthalten kann,
ein aromatischer C₆-C₃₀-Kohlenwasserstoffrest, der durch eine oder mehrere Substituenten substituiert sein kann, oder eine Kombination davon;
X -COO-, -CON(R¹)- oder entfällt, wobei die Bindung an A über O oder N erfolgt;
B ein cycloaliphatischer, linearer oder verzweigter aliphatischer C₁-C₃₀-Kohlenwasserstoffrest, der durch einen oder mehrere Substituenten substituiert sein kann, der durch eine oder mehrere Urethangruppen, Estergruppen, O und/oder S unterbrochen sein kann und der 1 bis 4 Benzolgruppen enthalten kann, ein aromatischer C₆-C₃₀-Kohlenwasserstoffrest, der durch einen oder mehrere Substituenten substituiert sein kann,
oder eine Kombination davon;
R¹ Wasserstoff ein cycloaliphatischer, linearer oder verzweigter aliphatischer C₁-C₁₀- Kohlenwasserstoffrest, der durch eine oder mehrere Sauerstoffatome unterbrochen sein kann und der durch eine oder mehrere OH-Gruppen substituiert sein kann, oder ein aromatischer C₆-C₁₀-Kohlenwasserstoffrest, der durch eine oder mehrere OH-Gruppen substituiert sein kann;
m eine ganze Zahl von 1 bis 6;
n eine ganze Zahl von 1 bis 6;
p eine ganze Zahl von 1 bis 6; wobei
m und p nicht gleichzeitig größer als 1 sein können und wobei dann, wenn m = 1 ist, p = n ist, und wenn p = 1 ist, m = n ist.

2. Dentalwerkstoff nach Anspruch 1, in dem der Sulfonsäureester eine Verbindung der Formel 2 ist, 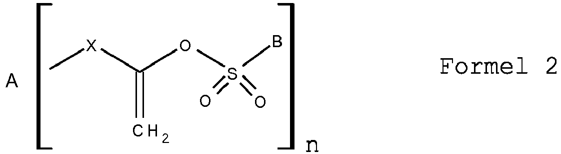 in der die Variablen vorzugsweise die folgenden Bedeutungen haben:
A H, CN, ein aliphatischer, linearer oder verzweigter C₁-C₃₀-Kohlenwasserstoffrest, der durch eine oder mehrere, vorzugsweise 1 bis 4, besonders bevorzugt 1 bis 2 Substituenten, die aus CH₃, -C₂H₅, -OH, -OCH₃ und/oder -O-COCH₃ ausgewählt sind, substituiert sein kann, der durch eine oder mehrere, vorzugsweise 1 bis 4, besonders bevorzugt 1 bis 2 Urethangruppen, Estergruppen, O und/oder S unterbrochen sein kann und der 1 bis 4, vorzugsweise 1 oder 2 Benzolgruppen, vorzugsweise 1,4-Phenylengruppen, enthalten kann, ein aromatischer C₆-C₃₀-Kohlenwasserstoffrest, der durch eine oder mehrere, vorzugsweise 1 bis 4, besonders bevorzugt 1 bis 2 Substituenten, die aus CH₃, -C₂H₅, -OH, -OCH₃ und/oder -O-COCH₃ ausgewählt sind, substituiert sein kann, oder eine Kombination davon;
X -COO-, -CON(R¹)- oder entfällt, wobei die Bindung an A über O oder N erfolgt und wobei X vorzugsweise entfällt, wenn A ein aromatischer Kohlenwasserstoffrest oder CN ist;
B ein aliphatischer, linearer oder verzweigter C₁-C₂₀-Kohlenwasserstoffrest, der durch einen oder mehrere, vorzugsweise 1 bis 3, besonders bevorzugt 1 bis 2 Substituenten, vorzugsweise C₁- bis C₅-Alkyl und/oder C₁- bis C₅-Alkoxy, substituiert sein kann, der durch eine oder mehrere, vorzugsweise 1 bis 4, besonders bevorzugt 1 bis 2 Urethangruppen, Estergruppen, O oder S unterbrochen sein kann und der 1 bis 4, vorzugsweise 1 oder 2 Benzolgruppen, vorzugweise 1,4-Phenylengruppen, enthalten kann,
ein aromatischer C₆-C₁₈-Kohlenwasserstoffrest, der durch einen oder mehrere, vorzugsweise 1 bis 3, besonders bevorzugt 1 bis 2 Substituenten, vorzugsweise C₁- bis C₅-Alkyl und/oder C₁- bis C₅-Alkoxy, substituiert sein kann, oder eine Kombination davon;
R¹ Wasserstoff oder ein linearer oder verzweigter aliphatischer C₁-C₁₀-Kohlenwasserstoffrest, der durch eine oder mehrere, vorzugsweise 1 bis 4, besonders bevorzugt 1 bis 2 Sauerstoffatome unterbrochen sein kann und der durch eine oder mehrere, vorzugsweise 1 bis 4, besonders bevorzugt 1 bis 2 OH-Gruppen substituiert sein kann;
n eine ganze Zahl von 1 bis 6.

3. Dentalwerkstoff nach Anspruch 2, in dem die Variablen der Formel 2 die folgenden Bedeutungen haben:
A ein aliphatischer linearer oder verzweigter C₁-C₂₀-Kohlenwasserstoffrest, der durch eine oder mehrere, vorzugsweise 1 bis 4, besonders bevorzugt 1 bis 2 1,4-Phenylengruppen, Urethangruppen, Estergruppen, 0 und/oder S unterbrochen sein kann und der durch eine oder mehrere, vorzugsweise 1 bis 4, besonders bevorzugt 1 bis 2 OH-Gruppen substituiert sein kann, oder ein Phenylrest, der durch einen oder mehrere, vorzugsweise 1 bis 3, besonders bevorzugt 1 bis 2 Substituenten, vorzugsweise -CH₃, und/oder -OCH₃ substituiert sein kann;
X -O-CO- oder entfällt, wobei die Bindung an A über O erfolgt und wobei X vorzugsweise entfällt, wenn A ein aromatischer Kohlenwasserstoffrest oder CN ist;
B ein aliphatischer linearer oder verzweigter C₁-C₂₀-Kohlenwasserstoffrest, der durch eine oder mehrere, vorzugsweise 1 bis 4, besonders bevorzugt 1 bis 2 Sauerstoffatome unterbrochen sein kann und der 1 bis 4, vorzugsweise 1 oder 2 Benzolgruppen, vorzugsweise 1,4-Phenylengruppen, enthalten kann, oder ein Phenylrest, der durch 1 oder 2, vorzugsweise 1 -OCH₃ oder vorzugsweise -CH₃ Gruppe(n) substituiert sein kann;
n 1 oder 2.

4. Dentalwerkstoff nach Anspruch 1, in dem der Sulfonsäureester eine Verbindung der Formel 3 ist, 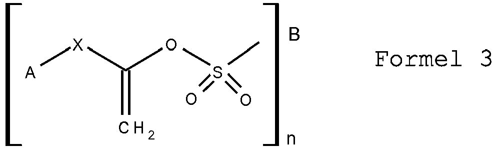 in der die Variablen vorzugsweise die folgenden Bedeutungen haben:
A Wasserstoff, -CN, ein Phenylrest, der durch einen oder mehrere, vorzugsweise 1 bis 3, besonders bevorzugt 1 bis 2 Substituenten, vorzugsweise -CH₃,-C₂H₅, -OH, -OCH₃ und/oder -O-COCH₃, substituiert sein kann, oder ein aliphatischer linearer oder verzweigter C₁-C₂₀-Kohlenwasserstoffrest, der durch eine oder mehrere, vorzugsweise 1 bis 4, besonders bevorzugt 1 bis 2 Urethangruppen, Estergruppen, O und/oder S unterbrochen sein kann, der 1 bis 4, vorzugsweise 1 oder 2 Benzolgruppen, vorzugsweise 1,4-Phenylengruppen, enthalten kann und der durch eine oder mehrere, vorzugsweise 1 bis 4, besonders bevorzugt 1 bis 2 OH-Gruppen substituiert sein kann;
B ein cycloaliphatischer, linearer oder verzweigter aliphatischer C₁-C₂₀-Kohlenwasserstoffrest, der durch eine oder mehrere, vorzugsweise 1 bis 4, besonders bevorzugt 1 bis 2 OH-Gruppen substituiert sein kann, der durch eine oder mehrere, vorzugsweise 1 bis 4, besonders bevorzugt 1 bis 2 Urethangruppen, Estergruppen, O und/oder S unterbrochen sein kann und der 1 bis 4, vorzugsweise 1 oder 2 Benzolgruppen enthalten kann,
ein aromatischer C₆-C₁₈-Kohlenwasserstoffrest, der durch eine oder mehrere, vorzugsweise 1 bis 4, besonders bevorzugt 1 bis 2 OH-Gruppen substituiert sein kann,
oder eine Kombination davon;
X -COO- oder -CON(R¹)- oder entfällt, wobei die Bindung an A über O oder N erfolgt und wobei X vorzugsweise entfällt, wenn A ein aromatischer Kohlenwasserstoffrest oder CN ist;
R¹ Wasserstoff oder ein linearer oder verzweigter aliphatischer C₁-C₁₀-Kohlenwasserstoffrest, der durch eine oder mehrere, vorzugsweise 1 bis 4, besonders bevorzugt 1 bis 2 Sauerstoffatome unterbrochen sein kann und der durch eine oder mehrere, vorzugsweise 1 bis 4, besonders bevorzugt 1 bis 2 OH-Gruppen substituiert sein kann;
n eine ganze Zahl von 2 bis 4.

5. Dentalwerkstoff nach Anspruch 4, in dem die Variablen der Formel 3 die folgenden Bedeutungen haben:
A H, ein Phenylrest, der durch einen oder mehrere Substituenten, vorzugsweise -CH₃, -OH, -OCH₃, substituiert sein kann, oder ein aliphatischer, linearer oder verzweigter C₁-C₈-Kohlenwasserstoffrest, der durch eine oder mehrere, vorzugsweise 1 bis 4, besonders bevorzugt 1 bis 2 Urethangruppen, Estergruppen und/oder O unterbrochen sein kann;
B ein aliphatischer, linearer oder verzweigter C₁-C₁₀-Kohlenwasserstoffrest, der durch eine oder mehrere, vorzugsweise 1 bis 4, besonders bevorzugt 1 bis 2 O-Atome unterbrochen sein kann und der 1 bis 4, vorzugsweise 1 oder 2 Benzolgruppen, vorzugsweise 1,4-Phenylengruppen, enthalten kann, ein aromatischer C₆-C₁₀-Kohlenwasserstoffrest, oder eine Kombination davon;
X -COO- oder entfällt, wobei die Bindung an A über O erfolgt und wobei X vorzugsweise entfällt, wenn A ein aromatischer Kohlenwasserstoffrest oder CN ist; und
n 2.

6. Dentalwerkstoff nach einem der Ansprüche 1 bis 5, der mindestens einen Füllstoff enthält.

7. Dentalwerkstoff nach einem der Ansprüche 1 bis 6, der
(a) 0,5 bis 40 Gew.-%, bevorzugt 1,0 bis 30 Gew.-% und besonders bevorzugt 2,0 bis 30 Gew.-% mindestens einer Verbindung der allgemeinen Formel 1,
(b) 0,01 bis 5,0 Gew.-%, bevorzugt 0,1 bis 5,0 Gew.-% und besonders bevorzugt 0,1 bis 3,0 Gew.-% Initiator(en) für die radikalische Polymerisation, und ggf.
(c) 5 bis 80 Gew.-%, bevorzugt 10 bis 70 Gew.-% und besonders bevorzugt 10 bis 60 Gew.-% multifunktionelle (Meth)acrylat(e) enthält,
jeweils bezogen auf die Gesamtmasse des Dentalwerkstoffs.

8. Dentalwerkstoff nach Anspruch 7 zur Verwendung als Prothesenmaterial, Adhäsiv oder Beschichtungsmaterial der
(a) 0,5 bis 40 Gew.-%, bevorzugt 1,0 bis 30 Gew.-% und besonders bevorzugt 2,0 bis 30 Gew.-% mindestens einer Verbindung der allgemeinen Formel 1,
(b) 0,01 bis 5,0 Gew.-%, bevorzugt 0,1 bis 5,0 Gew.-% und besonders bevorzugt 0,1 bis 3,0 Gew.-% Initiator(en),
(c) 5 bis 80 Gew.-%, bevorzugt 10 bis 80 Gew.-% und besonders bevorzugt 15 bis 80 Gew.-% multifunktionelle(s) (Meth)acrylat(e),
(d) 0 bis 40 Gew.-%, bevorzugt 0 bis 30 Gew.-% und besonders bevorzugt 0 bis 20 Gew.-% monofunktionelle(s) (Meth)acrylat(e),
(e) 0 bis 5 Gew.-%, bevorzugt 0 bis 3 Gew.-% und besonders bevorzugt 0,2 bis 3 Gew.-% Additiv(e).
(f) 0 bis 60 Gew.-%, bevorzugt 0 bis 50 Gew.-% und besonders bevorzugt 0 bis 40 Gew.-% Lösungsmittel, vorzugsweise Wasser, Ethanol und/oder Polyethylenglycol enthält,
jeweils bezogen auf die Gesamtmasse des Dentalwerkstoffs.

9. Dentalwerkstoff nach Anspruch 7 zur Verwendung als Komposit, Füllungskomposit oder Zement, der
(a) 0,5 bis 30 Gew.-%, bevorzugt 1,0 bis 30 Gew.-% und besonders bevorzugt 2,0 bis 20 Gew.-% mindestens einer Verbindung der allgemeinen Formel 1,
(b) 0,01 bis 5,0 Gew.-%, bevorzugt 0,1 bis 5,0 Gew.-% und besonders bevorzugt 0,1 bis 3,0 Gew.-% Initiator(en),
(c) 5 bis 50 Gew.-%, bevorzugt 10 bis 40 Gew.-% und besonders bevorzugt 10 bis 30 Gew.-% multifunktionelle(s) (Meth)acrylat(e),
(d) 0 bis 30 Gew.-%, bevorzugt 0 bis 20 Gew.-% und besonders bevorzugt 0 bis 10 Gew.-% monofunktionelle(s) (Meth)acrylat(e),
(e) 5 bis 85 Gew.-%, bevorzugt 20 bis 85 Gew.-% und besonders bevorzugt 30 bis 80 Gew.-% Füllstoff(e), und
(f) 0 bis 5 Gew.-%, bevorzugt 0 bis 3 Gew.-% und besonders bevorzugt 0,2 bis 3 Gew.-% Additiv(e) enthält,
jeweils bezogen auf die Gesamtmasse des Dentalwerkstoffs.

10. Dentalwerkstoff nach einem der Ansprüche 1 bis 9 zur intraoralen Verwendung zur Restauration geschädigter Zähne.

11. Verwendung eines Dentalwerkstoffs nach einem der Ansprüche 1 bis 9 als Material zur extraoralen Herstellung oder Reparatur von Dentalrestaurationen.

12. Verwendung nach Anspruch 11 zur Herstellung von künstlichen Zähnen, Prothesen, Inlays, Onlays, Kronen oder Brücken.

13. Verwendung eines Dentalwerkstoffs nach einem der Ansprüche 1 bis 9 zur Ex-vivo Herstellung von Formkörpern durch Gießen, Pressen oder 3D-Druck.

14. Verwendung einer Verbindung der Formel (1), in der die Variablen wie in Anspruch 1 oder einem der Ansprüche 2 bis 5 definiert sind, zur Verringerung der Polymerisationsschrumpfungsspannung und/oder zur Verbesserung der Schlagzähigkeit von Polymerisaten.

## Claims

1. Radically polymerisable dental material that comprises at least one multifunctional (meth)acrylate or a mixture of mono and multifunctional (meth)acrylates, at least one initiator for the radical polymerisation and at least one sulfonic acid ester of Formula 1: 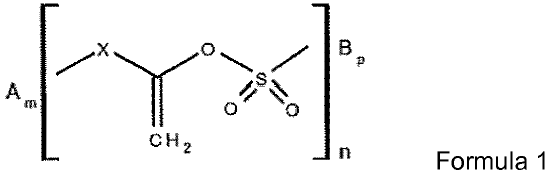 in which the variables have the following meanings:
A H, CN, a cycloaliphatic, linear or branched aliphatic C₁-C₃₀ hydrocarbon group, which can be substituted by one or more substituents, which can be interrupted by one or more urethane groups, ester groups, O and/or S and which can comprise 1 to 4 benzene groups, an aromatic C₆-C₃₀ hydrocarbon group that can be substituted by one or more substituents,
or a combination thereof;
X -COO-, -CON(R¹)- or is absent, wherein the bond to A is through O or N;
B a cycloaliphatic, linear or branched aliphatic C₁-C₃₀ hydrocarbon group, which can be substituted by one or more substituents, which can be interrupted by one or more urethane groups, ester groups, O and/or S and which can comprise 1 to 4 benzene groups,
an aromatic C₆-C₃₀ hydrocarbon group that can be substituted by one or more substituents,
or a combination thereof;
R¹ hydrogen, a cycloaliphatic, linear or branched aliphatic C₁-C₁₀ hydrocarbon group, which can be interrupted by one or more oxygen atoms and which can be substituted by one or more OH groups, or
an aromatic C₆-C₁₀ hydrocarbon group, which can be substituted by one or more OH groups;
m an integer from 1 to 6;
n an integer from 1 to 6;
p an integer from 1 to 6; wherein
m and p cannot simultaneously be greater than 1 and wherein, if m = 1, p = n, and if p = 1, m = n.

2. Dental material according to claim 1 in which the sulfonic acid ester is a compound of Formula 2, 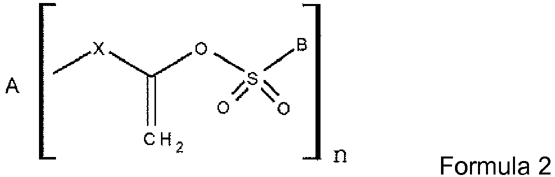 in which the variables preferably have the following meanings:
A H, CN, an aliphatic, linear or branched C₁-C₃₀ hydrocarbon group, which can be substituted by one or more, preferably 1 to 4, particularly preferably 1 to 2 substituents, which are selected from CH₃, -C₂H₅, -OH, -OCH₃ and/or -O-COCH₃, which can be interrupted by one or more, preferably 1 to 4, particularly preferably 1 to 2 urethane groups, ester groups, O and/or S and which can comprise 1 to 4, preferably 1 or 2 benzene groups, preferably 1,4-phenylene groups,
an aromatic C₆-C₃₀ hydrocarbon group, which can be substituted by one or more, preferably 1 to 4, particularly preferably 1 to 2 substituents, which are selected from CH₃, -C₂H₅, -OH, -OCH₃ and/or O-COCH₃,
or a combination thereof;
X -COO-, -CON(R¹)- or is absent, wherein the bond to A is through O or N and wherein X is preferably absent if A is an aromatic hydrocarbon group or CN;
B an aliphatic, linear or branched C₁-C₂₀ hydrocarbon group, which can be substituted by one or more, preferably 1 to 3, particularly preferably 1 to 2 substituents, preferably C₁ to C₅ alkyl and/or C₁ to C₅ alkoxy, which can be interrupted by one or more, preferably 1 to 4, particularly preferably 1 to 2 urethane groups, ester groups, O or S and which can comprise 1 to 4, preferably 1 or 2 benzene groups, preferably 1,4-phenylene groups, an aromatic C₆-C₁₈ hydrocarbon group, which can be substituted by one or more, preferably 1 to 3, particularly preferably 1 to 2 substituents, preferably C₁ to C₅ alkyl and/or C₁ to C₅ alkoxy, or a combination thereof;
R¹ hydrogen or a linear or branched aliphatic C₁-C₁₀ hydrocarbon group, which can be interrupted by one or more, preferably 1 to 4, particularly preferably 1 to 2 oxygen atoms and which can be substituted by one or more, preferably 1 to 4, particularly preferably 1 to 2 OH groups;
n an integer from 1 to 6.

3. Dental material according to claim 2, in which the variables of Formula 2 have the following meanings:
A an aliphatic linear or branched C₁-C₂₀ hydrocarbon group, which can be interrupted by one or more, preferably 1 to 4, particularly preferably 1 to 2 1,4-phenylene groups, urethane groups, ester groups, O and/or S and which can be substituted by one or more, preferably 1 to 4, particularly preferably 1 to 2 OH groups, or a phenyl group, which can be substituted by one or more, preferably 1 to 3, particularly preferably 1 to 2 substituents, preferably -CH₃, and/or -OCH₃;
X -O-CO- or is absent, wherein the bond to A is through O and wherein X is preferably absent if A is an aromatic hydrocarbon group or CN;
B an aliphatic linear or branched C₁-C₂₀ hydrocarbon group, which can be interrupted by one or more, preferably 1 to 4, particularly preferably 1 to 2 oxygen atoms and which can comprise 1 to 4, preferably 1 or 2 benzene groups, preferably 1,4-phenylene groups, or a phenyl group, which can be substituted by 1 or 2, preferably 1 -OCH₃ or preferably -CH₃ group(s);
n 1 or 2.

4. Dental material according to claim 1 in which the sulfonic acid ester is a compound of Formula 3, 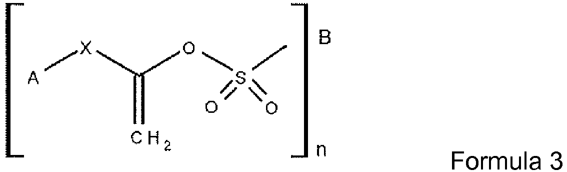 in which the variables preferably have the following meanings:
A hydrogen, -CN, a phenyl group, which can be substituted by one or more, preferably 1 to 3, particularly preferably 1 to 2 substituents, preferably -CH₃, -C₂H₅, -OH, -OCH₃ and/or -O-COCH₃, or an aliphatic linear or branched C₁-C₂₀ hydrocarbon group, which can be interrupted by one or more, preferably 1 to 4, particularly preferably 1 to 2 urethane groups, ester groups, O and/or S, which can contain 1 to 4, preferably 1 or 2 benzene groups, preferably 1,4-phenylene groups and which can be substituted by one or more, preferably 1 to 4, particularly preferably 1 to 2 OH groups;
B a cycloaliphatic, linear or branched aliphatic C₁-C₂₀ hydrocarbon group, which can be substituted by one or more, preferably 1 to 4, particularly preferably 1 to 2 OH groups, which can be interrupted by one or more, preferably 1 to 4, particularly preferably 1 to 2 urethane groups, ester groups, O and/or S and which can contain 1 to 4, preferably 1 or 2 benzene groups, an aromatic C₆-C₁₈ hydrocarbon group, which can be substituted by one or more, preferably 1 to 4, particularly preferably 1 to 2 OH groups,
or a combination thereof;
X -COO- or -CON(R¹)- or is absent, wherein the bond to A is through O or N and wherein X is preferably absent if A is an aromatic hydrocarbon group or CN;
R¹ hydrogen or a linear or branched aliphatic C₁-C₁₀ hydrocarbon group, which can be interrupted by one or more, preferably 1 to 4, particularly preferably 1 to 2 oxygen atoms and which can be substituted by one or more, preferably 1 to 4, particularly preferably 1 to 2 OH groups;
n an integer from 2 to 4.

5. Dental material according to claim 4, in which the variables of Formula 3 have the following meanings:
A H, a phenyl group, which can be substituted by one or more substituents, preferably -CH₃, -OH, -OCH₃, or an aliphatic, linear or branched C₁-C₈ hydrocarbon group, which can be interrupted by one or more, preferably 1 to 4, particularly preferably 1 to 2 urethane groups, ester groups and/or O;
B an aliphatic, linear or branched C₁-C₁₀ hydrocarbon group, which can be interrupted by one or more, preferably 1 to 4, particularly preferably 1 to 2 O atoms and which can comprise 1 to 4, preferably 1 or 2 benzene groups, preferably 1,4-phenylene groups,
an aromatic C₆-C₁₀ hydrocarbon group,
or a combination thereof;
X -COO- or is absent, wherein the bond to A is through O and wherein X is preferably absent if A is an aromatic hydrocarbon group or CN; and
n 2.

6. Dental material according to one of claims 1 to 5 which comprises at least one filler.

7. Dental material according to one of claims 1 to 6 which comprises
(a) 0.5 to 40 wt %, preferably 1.0 to 30 wt % and particularly preferably 2.0 to 30 wt % of at least one compound of the General Formula 1,
(b) 0.01 to 5.0 wt %, preferably 0.1 to 5.0 wt % and particularly preferably 0.1 to 3.0 wt % initiator(s) for the radical polymerisation, and optionally
(c) 5 to 80 wt %, preferably 10 to 70 wt % and particularly preferably 10 to 60 wt % multifunctional (meth)acrylate(s),
in each case relative to the total mass of the dental material.

8. Dental material according to claim 7 for use as a prosthesis material, adhesive or coating material which comprises
(a) 0.5 to 40 wt %, preferably 1.0 to 30 wt % and particularly preferably 2.0 to 30 wt % of at least one compound of the General Formula 1,
(b) 0.01 to 5.0 wt %, preferably 0.1 to 5.0 wt % and particularly preferably 0.1 to 3.0 wt % initiator(s),
(c) 5 to 80 wt %, preferably 10 to 80 wt % and particularly preferably 15 to 80 wt % multifunctional (meth)acrylate(s),
(d) 0 to 40 wt %, preferably 0 to 30 wt % and particularly preferably 0 to 20 wt % monofunctional (meth)acrylate(s),
(e) 0 to 5 wt %, preferably 0 to 3 wt % and particularly preferably 0.2 to 3 wt % additive(s),
(f) 0 to 60 wt %, preferably 0 to 50 wt % and particularly preferably 0 to 40 wt % solvent, preferably water, ethanol and/or polyethylene glycol,
in each case relative to the total mass of the dental material.

9. Dental material according to claim 7 for use as a composite, filling composite or cement, which comprises
(a) 0.5 to 30 wt %, preferably 1.0 to 30 wt % and particularly preferably 2.0 to 20 wt % of at least one compound of the General Formula 1,
(b) 0.01 to 5.0 wt %, preferably 0.1 to 5.0 wt % and particularly preferably 0.1 to 3.0 wt % initiator(s),
(c) 5 to 50 wt %, preferably 10 to 40 wt % and particularly preferably 10 to 30 wt % multifunctional (meth)acrylate(s),
(d) 0 to 30 wt %, preferably 0 to 20 wt % and particularly preferably 0 to 10 wt % monofunctional (meth)acrylate(s),
(e) 5 to 85 wt %, preferably 20 to 85 wt % and particularly preferably 30 to 80 wt % filler(s), and
(f) 0 to 5 wt %, preferably 0 to 3 wt % and particularly preferably 0.2 to 3 wt % additive(s),
in each case relative to the total mass of the dental material.

10. Dental material according to one of claims 1 to 9 for intraoral use to restore damaged teeth.

11. Use of a dental material according to one of claims 1 to 9 as a material for the extraoral manufacture or repair of dental restorations.

12. Use according to claim 11 for the manufacture of artificial teeth, prostheses, inlays, onlays, crowns or bridges.

13. Use of a dental material according to one of claims 1 to 9 for the ex-vivo manufacture of mouldings by casting, compression moulding or 3D printing.

14. Use of a compound of Formula (1), in which the variables are defined as in claim 1 or one of claims 2 to 5, to reduce the polymerisation shrinkage stress and/or to improve the impact strength of polymers.

## Revendications

1. Matériau dentaire polymérisable par voie radicalaire, qui comporte au moins un acrylate ou méthacrylate polyfonctionnel ou un mélange d'acrylates ou méthacrylates monofonctionnels et polyfonctionnels, au moins un amorceur de polymérisation radicalaire et au moins un ester sulfonate de formule 1 : 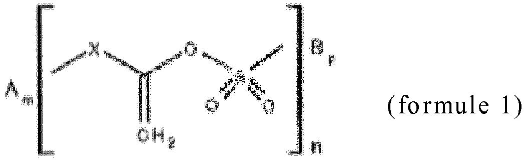 dans laquelle les symboles ont les significations suivantes :
- A représente un atome d'hydrogène, un groupe cyano,
un reste d'hydrocarbure aliphatique linéaire ou ramifié ou cycloaliphatique en C₁-C₃₀, qui peut porter un ou plusieurs substituant(s), être interrompu par un ou plusieurs groupe(s) de type uréthane et/ou ester et/ou atome(s) d'oxygène et/ou de soufre, et comporter de 1 à 4 groupe(s) benzénique(s),
un reste d'hydrocarbure aromatique en C₆-C₃₀, qui peut porter un ou plusieurs substituant(s),
ou une combinaison de telles entités ;
- X représente un raccord de formule -COO- ou -CON(R¹)- dont la liaison à l'entité A est assurée par l'atome d'oxygène ou d'azote, ou ne représente rien ;
- B représente un reste d'hydrocarbure aliphatique linéaire ou ramifié ou cycloaliphatique en C₁-C₃₀, qui peut porter un ou plusieurs substituant(s), être interrompu par un ou plusieurs groupe(s) de type uréthane et/ou ester et/ou atome(s) d'oxygène et/ou de soufre, et comporter de 1 à 4 groupe(s) benzénique(s),
un reste d'hydrocarbure aromatique en C₆-C₃₀, qui peut porter un ou plusieurs substituant(s),
ou une combinaison de telles entités ;
- R¹ représente un atome d'hydrogène,
un reste d'hydrocarbure aliphatique linéaire ou ramifié ou cycloaliphatique en C₁-C₁₀, qui peut être interrompu par un ou plusieurs atome(s) d'oxygène, et porter un ou plusieurs substituant(s) hydroxyle ;
ou un reste d'hydrocarbure aromatique en C₆-C₁₀, qui peut porter un ou plusieurs substituant(s) hydroxyle ;
- l'indice m est un nombre entier valant de 1 à 6 ;
- l'indice n est un nombre entier valant de 1 à 6 ;
- et l'indice p est un nombre entier valant de 1 à 6 ;
étant entendu que les indices m et p ne peuvent pas être simultanément plus grands que 1, et que si m = 1, alors p = n, et si p = 1, alors m = n.

2. Matériau dentaire conforme à la revendication 1, dans lequel l'ester sulfonate est un composé de formule 2 : 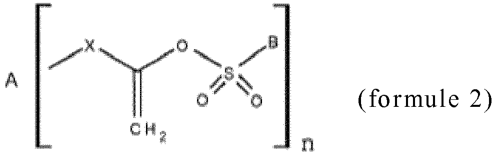 dans laquelle formule les symboles ont de préférence les significations suivantes :
- A représente un atome d'hydrogène, un groupe cyano,
un reste d'hydrocarbure aliphatique linéaire ou ramifié ou cycloaliphatique en C₁-C₃₀, qui peut porter un ou plusieurs, de préférence de 1 à 4 et mieux encore 1 ou 2 substituant(s), choisi(s) parmi les groupes méthyle, éthyle, hydroxyle, méthoxy et/ou acétoxy, être interrompu par un ou plusieurs, de préférence de 1 à 4 et mieux encore 1 ou 2 groupe(s) de type uréthane et/ou ester et/ou atome(s) d'oxygène et/ou de soufre, et comporter de 1 à 4 et de préférence 1 ou 2 groupe(s) benzénique(s), de préférence 1,4-phénylène,
un reste d'hydrocarbure aromatique en C₆-C₃₀, qui peut porter un ou plusieurs, de préférence de 1 à 4 et mieux encore 1 ou 2 substituant(s), choisi(s) parmi les groupes méthyle, éthyle, hydroxyle, méthoxy et/ou acétoxy,
ou une combinaison de telles entités ;
- X représente un raccord de formule -COO- ou -CON(R¹)- dont la liaison à l'entité A est assurée par l'atome d'oxygène ou d'azote, ou ne représente rien, étant entendu que, de préférence, X ne représente rien si A représente un reste d'hydrocarbure aromatique ou un groupe cyano ;
- B représente un reste d'hydrocarbure aliphatique linéaire ou ramifié ou cycloaliphatique en C₁-C₂₀, qui peut porter un ou plusieurs, de préférence de 1 à 3 et mieux encore 1 ou 2 substituant(s), de préférence alkyle en C₁-C₅ et/ou alcoxy en C₁-C₅, être interrompu par un ou plusieurs, de préférence de 1 à 4 et mieux encore 1 ou 2 groupe(s) de type uréthane ou ester ou atome(s) d'oxygène ou de soufre, et comporter de 1 à 4 et de préférence 1 ou 2 groupe(s) benzénique(s), de préférence 1,4-phénylène,
un reste d'hydrocarbure aromatique en C₆-C₁₈, qui peut porter un ou plusieurs, de préférence de 1 à 3 et mieux encore 1 ou 2 substituant(s), de préférence alkyle en C₁-C₅ et/ou alcoxy en C₁-C₅,
ou une combinaison de telles entités ;
- R¹ représente un atome d'hydrogène,
ou un reste d'hydrocarbure aliphatique linéaire ou ramifié en C₁-C₁₀, qui peut être interrompu par un ou plusieurs, de préférence de 1 à 4 et mieux encore 1 ou 2 atome(s) d'oxygène, et porter un ou plusieurs, de préférence de 1 à 4 et mieux encore 1 ou 2 substituant(s) hydroxyle ;
- et l'indice n est un nombre entier valant de 1 à 6.

3. Matériau dentaire conforme à la revendication 2, dans lequel les symboles utilisés dans la formule 2 ont les significations suivantes :
- A représente un reste d'hydrocarbure aliphatique linéaire ou ramifié en C₁-C₂₀, qui peut être interrompu par un ou plusieurs, de préférence de 1 à 4 et mieux encore 1 ou 2 groupe(s) 1,4-phénylène, groupe(s) de type uréthane et/ou ester et/ou atome(s) d'oxygène et/ou de soufre, et porter un ou plusieurs, de préférence de 1 à 4 et mieux encore 1 ou 2 substituant(s) hydroxyle,
ou un groupe phényle qui peut porter un ou plusieurs, de préférence de 1 à 3 et mieux encore 1 ou 2 substituant(s), de préférence méthyle et/ou méthoxy ;
- X représente un raccord de formule -O-CO- dont la liaison à l'entité A est assurée par l'atome d'oxygène, ou ne représente rien, étant entendu que, de préférence, X ne représente rien si A représente un reste d'hydrocarbure aromatique ou un groupe cyano ;
- B représente un reste d'hydrocarbure aliphatique linéaire ou ramifié en C₁-C₂₀, qui peut être interrompu par un ou plusieurs, de préférence de 1 à 4 et mieux encore 1 ou 2 atome(s) d'oxygène, et comporter de 1 à 4 et de préférence 1 ou 2 groupe(s) benzénique(s), de préférence 1,4-phénylène,
ou un groupe phényle qui peut porter 1 ou 2, de préférence 1, substituant(s) méthoxy ou de préférence méthyle ;
- et l'indice n vaut 1 ou 2.

4. Matériau dentaire conforme à la revendication 1, dans lequel l'ester sulfonate est un composé de formule 3 : 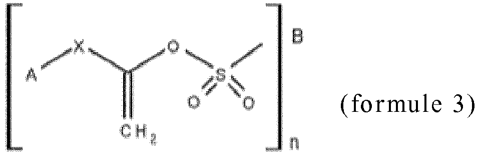 dans laquelle formule les symboles ont de préférence les significations suivantes :
- A représente un atome d'hydrogène, un groupe cyano,
un groupe phényle qui peut porter un ou plusieurs, de préférence de 1 à 3 et mieux encore 1 ou 2 substituant(s), de préférence méthyle, éthyle, hydroxyle, méthoxy et/ou acétoxy,
ou un reste d'hydrocarbure aliphatique linéaire ou ramifié en C₁-C₂₀, qui peut être interrompu par un ou plusieurs, de préférence de 1 à 4 et mieux encore 1 ou 2 groupe(s) de type uréthane et/ou ester et/ou atome(s) d'oxygène et/ou de soufre, comporter de 1 à 4 et de préférence 1 ou 2 groupe(s) benzénique(s), de préférence 1,4-phénylène, et porter un ou plusieurs, de préférence de 1 à 4 et mieux encore 1 ou 2 substituant(s) hydroxyle ;
- B représente un reste d'hydrocarbure aliphatique linéaire ou ramifié ou cycloaliphatique en C₁-C₂₀, qui peut porter un ou plusieurs, de préférence de 1 à 4 et mieux encore 1 ou 2 substituant(s) hydroxyle, être interrompu par un ou plusieurs, de préférence de 1 à 4 et mieux encore 1 ou 2 groupe(s) de type uréthane et/ou ester et/ou atome(s) d'oxygène et/ou de soufre, et comporter de 1 à 4 et de préférence 1 ou 2 groupe(s) benzénique(s),
un reste d'hydrocarbure aromatique en C₆-C₁₈, qui peut porter un ou plusieurs, de préférence de 1 à 4 et mieux encore 1 ou 2 substituant(s) hydroxyle,
ou une combinaison de telles entités ;
- X représente un raccord de formule -COO- ou -CON(R¹)- dont la liaison à l'entité A est assurée par l'atome d'oxygène ou d'azote, ou ne représente rien, étant entendu que, de préférence, X ne représente rien si A représente un reste d'hydrocarbure aromatique ou un groupe cyano ;
- R¹ représente un atome d'hydrogène,
ou un reste d'hydrocarbure aliphatique linéaire ou ramifié en C₁-C₁₀, qui peut être interrompu par un ou plusieurs, de préférence de 1 à 4 et mieux encore 1 ou 2 atome(s) d'oxygène, et porter un ou plusieurs, de préférence de 1 à 4 et mieux encore 1 ou 2 substituant(s) hydroxyle ;
- et l'indice n est un nombre entier valant de 2 à 4.

5. Matériau dentaire conforme à la revendication 4, dans lequel les symboles utilisés dans la formule 3 ont les significations suivantes :
- A représente un atome d'hydrogène,
un groupe phényle qui peut porter un ou plusieurs substituant(s),
de préférence méthyle, hydroxyle ou méthoxy,
ou un reste d'hydrocarbure aliphatique linéaire ou ramifié en C₁-C₈, qui peut être interrompu par un ou plusieurs, de préférence de 1 à 4 et mieux encore 1 ou 2 groupe(s) de type uréthane et/ou ester et/ou atome(s) d'oxygène ;
- B représente un reste d'hydrocarbure aliphatique linéaire ou ramifié en C₁-C₁₀, qui peut être interrompu par un ou plusieurs, de préférence de 1 à 4 et mieux encore 1 ou 2 atome(s) d'oxygène, et comporter de 1 à 4 et de préférence 1 ou 2 groupe(s) benzénique(s),
de préférence 1,4-phénylène,
un reste d'hydrocarbure aromatique en C₆-C₁₀,
ou une combinaison de telles entités ;
- X représente un raccord de formule -COO- dont la liaison à l'entité A est assurée par l'atome d'oxygène, ou ne représente rien, étant entendu que, de préférence, X ne représente rien si A représente un reste d'hydrocarbure aromatique ou un groupe cyano ;
- et l'indice n vaut 2.

6. Matériau dentaire conforme à l'une des revendications 1 à 5, qui contient au moins une charge.

7. Matériau dentaire conforme à l'une des revendications 1 à 6, qui contient :
a) de 0,5 à 40 % en poids, de préférence de 1,0 à 30 % en poids et mieux encore de 2,0 à 30 % en poids d'au moins un composé de formule générale 1,
b) de 0,01 à 5,0 % en poids, de préférence de 0,1 à 5,0 % en poids et mieux encore de 0,1 à 3,0 % en poids d'amorceur(s) de polymérisation, radicalaire,
c) et en option, de 5 à 80 % en poids, de préférence de 10 à 70 % en poids et mieux encore de 10 à 60 % en poids d'acrylate(s) ou méthacrylate(s) polyfonctionnel(s),
dans chaque cas par rapport au poids total du matériau dentaire.

8. Matériau dentaire conforme à la revendication 7 pour utilisation en tant que matériau de prothèse, adhésif ou matériau de revêtement, qui contient :
a) de 0,5 à 40 % en poids, de préférence de 1,0 à 30 % en poids et mieux encore de 2,0 à 30 % en poids d'au moins un composé de formule générale 1,
b) de 0,01 à 5,0 % en poids, de préférence de 0,1 à 5,0 % en poids et mieux encore de 0,1 à 3,0 % en poids d'amorceur(s),
c) de 5 à 80 % en poids, de préférence de 10 à 80 % en poids et mieux encore de 15 à 80 % en poids d'acrylate(s) ou méthacrylate(s) polyfonctionnel(s),
d) de 0 à 40 % en poids, de préférence de 0 à 30 % en poids et mieux encore de 0 à 20 % en poids d'acrylate(s) ou méthacrylate(s) mono fonctionnel(s),
e) de 0 à 5 % en poids, de préférence de 0 à 3 % en poids et mieux encore de 0,2 à 3 % en poids d'adjuvant(s),
f) de 0 à 60 % en poids, de préférence de 0 à 50 % en poids et mieux encore de 0 à 40 % en poids de solvant, de préférence d'eau, d'éthanol et/ou de polyéthylèneglycol,
dans chaque cas par rapport au poids total du matériau dentaire.

9. Matériau dentaire conforme à la revendication 7 pour utilisation en tant que composite, charge composite ou ciment, qui contient :
a) de 0,5 à 30 % en poids, de préférence de 1,0 à 30 % en poids et mieux encore de 2,0 à 20 % en poids d'au moins un composé de formule générale 1,
b) de 0,01 à 5,0 % en poids, de préférence de 0,1 à 5,0 % en poids et mieux encore de 0,1 à 3,0 % en poids d'amorceur(s),
c) de 5 à 50 % en poids, de préférence de 10 à 40 % en poids et mieux encore de 10 à 30 % en poids d'acrylate(s) ou méthacrylate(s) polyfonctionnel(s),
d) de 0 à 30 % en poids, de préférence de 0 à 20 % en poids et mieux encore de 0 à 10 % en poids d'acrylate(s) ou méthacrylate(s) mono fonctionnel(s),
e) de 5 à 85 % en poids, de préférence de 20 à 85 % en poids et mieux encore de 30 à 80 % en poids de charge(s),
f) et de 0 à 5 % en poids, de préférence de 0 à 3 % en poids et mieux encore de 0,2 à 3 % en poids d'adjuvant(s),
dans chaque cas par rapport au poids total du matériau dentaire.

10. Matériau dentaire, conforme à l'une des revendications 1 à 9, pour utilisation intrabuccale pour restauration de dents abimées.

11. Utilisation d'un matériau dentaire, conforme à l'une des revendications 1 à 9, en tant que matériau pour fabrication ou réparation extrabuccale de restaurations dentaires.

12. Utilisation conforme à la revendication 11 pour la fabrication de dents artificielles, de prothèses, d'incrustations inlay ou onlay, de couronnes ou de bridges.

13. Utilisation d'un matériau dentaire, conforme à l'une des revendications 1 à 9, pour la fabrication de corps moulés par coulée, compression ou impression-3D.

14. Utilisation d'un composé de formule (1), dans laquelle les symboles ont les significations indiquées dans la revendication 1 ou dans l'une des revendications 2 à 5, pour faire baisser la tension due au retrait de polymérisation et/ou pour améliorer la résistance au choc de matériaux résultant d'une polymérisation.
